(19) Europäisches Patentamt
European Patent Office
Office européen des brevets

(11) Numéro de publication: **0 105 776 B1**

(12) **FASCICULE DE BREVET EUROPEEN**

(45) Date de publication du fascicule du brevet: **26.02.86**

(21) Numéro de dépôt: **83401804.6**

(22) Date de dépôt: **15.09.83**

(51) Int. Cl.[4]: **C 07 D 498/06**, A 61 K 31/55 // C07D209/94 ,(C07D498/06, 265:00, 209:00)

(54) **Nouveaux dérivés de la C-homo 9-oxaergoline, leurs sels, procédé de préparation et intermédiaires de préparation, application à titre de médicaments et compositions les renfermant.**

(30) Priorité: **20.09.82 FR 8215773**

(43) Date de publication de la demande: **18.04.84 Bulletin 84/16**

(45) Mention de la délivrance du brevet: **26.02.86 Bulletin 86/9**

(84) Etats contractants désignés: **AT BE CH DE FR GB IT LI LU NL SE**

(56) Documents cités:
**EP - A - 0 033 767**
**EP - A - 0 034 546**

(73) Titulaire: **ROUSSEL-UCLAF, 35, boulevard des Invalides, F-75007 Paris (FR)**

(72) Inventeur: **Nedelec, Lucien, 45, boulevard de l'Ouest, F-93340 Le Raincy (FR)**
Inventeur: **Gasc, Jean Claude, 6 Place Georges Lyssandre, F-93140 Bondy (FR)**
Inventeur: **Fauveau, Patrick, 40 Avenue Camille Desmoulins, F-93190 Livry-Gargan (FR)**

(74) Mandataire: **Vieillefosse, Jean-Claude et al, ROUSSEL-UCLAF Boîte postale no. 9 111, route de Noisy, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



LIBER, STOCKHOLM 1986

**Description**

La présente invention concerne de nouveaux dérivés de la C-homo 9-oxaergoline ainsi que leurs sels, le procédé et les intermédiaires de préparation, l'application ü titre de médicaments de ces nouveaux dérivés, et les compositions les renfermant.

Des dérivés du cycloheptindole ayant entre autres des proprietés agonistes clopaminergiques et antiulcères sont décrits dans EP-A- 34546.

L'invention a pour objet de nouveaux dérivés de la Chomo 9-oxaergoline, ainsi que leurs sels d'addition avec les acides minéraux ou organiques, caractérisés en ce qu'ils repondent à la formule générale (I):

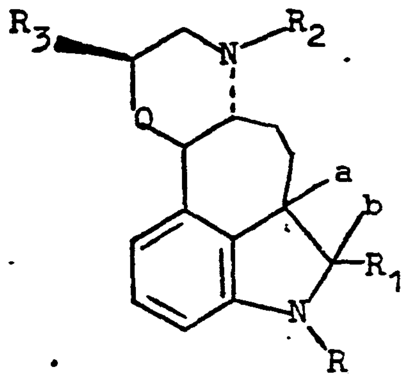

(I)

dans laquelle R représente un atome d'hydrogène ou un radical alcoyle renfermant de 1 à 4 atomes de carbone, $R_1$ représente un atome d'hydrogène, de chlore ou de brome ou forme avec b un radical oxo, a forme avec b une double liaison ou a représente un atome d'hydrogène quand b forme avec $R_1$ un ràdical oxo, b forme avec a une double liaison ou forme avec $R_1$ un radical oxo, $R_2$ représente un atome d'hydrogène, un radical alcoyle renfermant de 1 à 4 atomes de carbone, aralcoyle renfermant de 7 à 12 atomes de carbone, ou cycloalcoylalcoyle renfermant de 4 à 7 atomes de carbone, $R_3$ représente un radical hydroxyméthyle, alcoylthiométhyle, cyanométhyle, ou carboxy éventuellement estérifié par un alcool aliphatique renfermant de 1 à 5 atomes de carbone, ou amidifié par une amine de formule

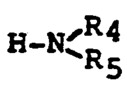

dans laquelle $R_4$ représente un atome d'hydrogène ou un radical alcoyle renfermant de 1 à 4 atomes de carbone, et $R_5$ représente un radical alcoyle renfermant de 1 à 4-atomes de carbone ou $R_4$ et $R_5$ forment ensemble avec l'atome d'azote un hétérocycle saturé renfermant éventuellement un autre hétéroatome.

Dans la formule générale (I) et dans ce qui suit, le terme radical alcoyle renfermant de 1 à 4 atomes de carbone désigne de préférence un radical méthyle, éthyle, propyle ou isopropyle; le terme alcool aliphatique renfermant de 1 à 5 atomes de carbone désigne de préférence le méthanol, l'éthanol, le propanol ou l'isopropanol; le terme radical aralcoyle renfermant de 7 à 12 atomes de carbone désigne de préférence un radical benzyle ou phénéthyle éventuellement substitué; le terme radical cycloalcoylalcoyle désigne, de préférence, un radical cyclopropylméthyle; le terme alcoyl-thiométhyle désigne par exemple le radical n-propyl thiométhyle, éthylthiométhyle ou de préférence méthylthiométhyle; le cycle éventuellement formé par $R_4$ et $R_5$ avec l'atome d'azote est par exemple, un cycle pyrrolidino, pipéridino, morpholino ou pipérazino; lorsque l'atome d'azote forme avec $R_4$ et $R_5$ un hétérocycle saturé renfermant un autre atome d'azote, ce dernier peut être substitué par un radical alcoyle renfermant de 1 à 4 atomes de carbone. Le pointillé signifie que la jonction entre le cycle morpholino et le cycle benzocycloheptène est de configuration trans. Bien entendu, les racémiques ainsi que les isomères optiquement actifs entrent dans le cadre de l'invention.

Les sels d'addition avec les acides minéraux ou organiques peuvent être par exemple, les sels formés avec les acides chlorhydrique, bromhydrique, nitrique, sulfurique, phosphorique, acétique, formique, propionique, benzoîque, maléique, fumarique, succinique, tartrique, citrique, oxalique, glyoxylique, aspartique, alcanesulfoniques tels que les acides méthane ou éthane sulfoniques, arylsulfoniques tels que acides benzèn$_e$ ou paratoluènesulfoniques et arylcarboxyliques.

Parmi les produits objet de l'invention, on peut citer notamment les dérivés répondant à la formule (I) ci-dessus, ainsi que leurs sels d'addition avec les acides minéraux ou organiques, caractérisés en ce que, dans ladite formule (I), à et b forment une double liaison et notamment parmi ceux-ci, ceux dans lesquels R représente un atome d'hydrogène.

Parmi ces derniers, on peut citer tout particulièrement les dérivés caractérisés en ce que dans ladite formule (I), $R_1$ représente un atome d'hydrogène, de chlore ou de brome, $R_2$ represente un radical alcoyle renfermant de 1 à 4 atomes de carbone et $R_3$ représente un radical hydroxyméthyle, un radical méthylthiométhyle, cyanométhyle ou un radical carboxy éventuellement estérifié par un alcool aliphatique linéaire renfermant de 1 à 4 atomes de carbone, ou amidifié par une amine de formule

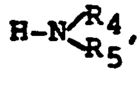,

dans laquelle $R_4$ et $R_5$ représentent un radical alcoyle linéaire renfermant de 1 à 4 atomes de carbone et tout particulièrement:

- le / 5 RS (5α, 8β, 10β)/ 6-méthyl C-homo 9-oxaergoline-8-carboxylate d'éthyle
- le / 5 RS (5α, 8β, 10β)/ 6-méthyl C-homo 9-oxaergoline-8-carboxylate de méthyle et
- le / 5 RS (5α, 8β, 10β)/ 6-méthyl C-homo 9-oxaergoline-8-méthanol,

ainsi que leurs sels d'addition avec les acides minéraux ou organiques.

On peut citer également le:

- / 5 RS (5α, 8β, 10β)/ 6-méthyl C-homo 9-oxaergoline-8-acétonitrile,
- l'acide / 5 RS (5α, 8β, 10β)/ 6-méthyl C-homo 9-oxaer-goline-8-carboxylique, ainsi que les produits non cités cidessus, figurant dans la partie expérimentale.

L'invention a également pour objet un procédé de préparation des dérivés, tels que définis par la formule I cidessus, ainsi que de leurs sels, caractérisés en ce que l'on cyclise un produit de formule II:

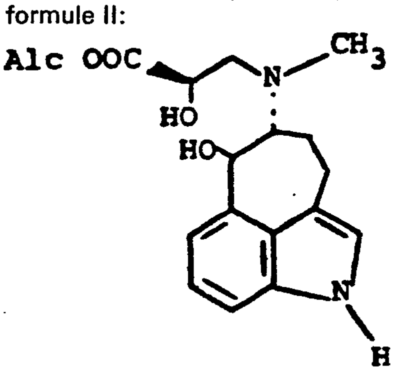

(II)

dans laquelle Alc représente un radical alcoyle renfermant de 1 à 4 atomes de carbone, pour obtenir un produit de formule (III):

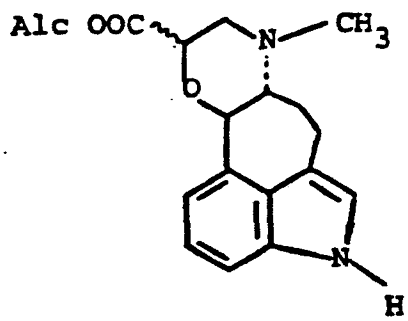

(III)

dans laquelle Alc a la signification déjà indiquée, et le trait ondulé signifie que le substituant est en position 8αou 8β, dont, soit l'on isole l'isomère 8β, soit l'on épimérise l'isomère 8α, puis estérifie l'acide ainsi obtenu dans lequel le substituant en 8 est en position 8β, pour obtenir un produit de formule ($I_A$):

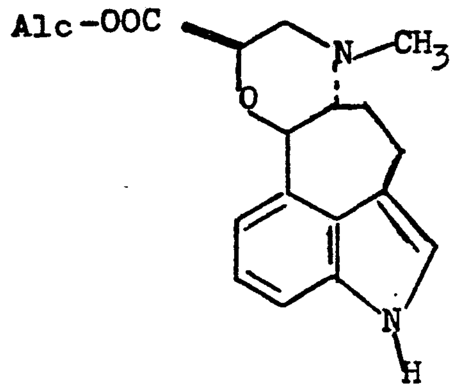

($I_A$)

dans laquelle Alc a la signification déjà indiquée, que

- <u>soit</u> l'on isole et si désiré, salifie,
- <u>soit</u> l'on déméthyle pour obtenir un produit de formule ($I_B$):

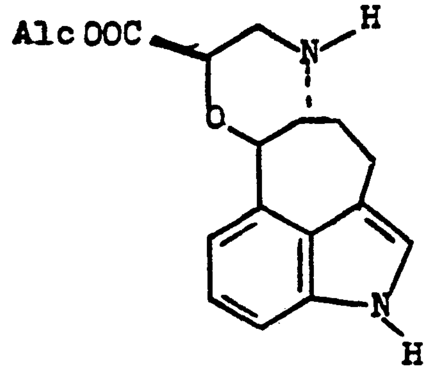

($I_B$)

dans laquelle Alc a la signification déjà indiquée, que soit l'on isole et si désiré salifie, soit l'on fait réagir avec un agent d'alcoylation pour obtenir un produit de formule ($I_C$):

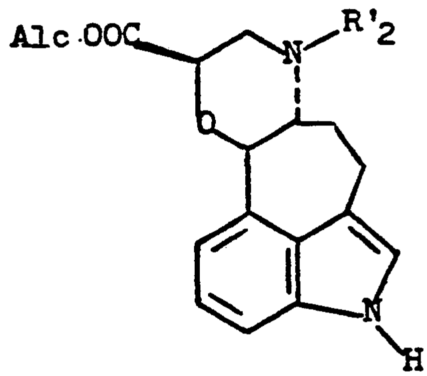

($I_C$)

dans laquelle Alc a la signification déjà indiquée, et $R'_2$ a la signification de $R_2$ déjà indiquée à l'exception de l'hydrogène, que <u>ou bien</u> l'on isole, et, si désiré, salifie, <u>ou bien</u> l'on fait réagir avec un amidure alcalin, puis avec un halogénure d'alcoyle de formule (IV):

Hal-R' (IV)

dans laquelle Hal représente un atome de chlore, de brome ou d'iode et R' représente un radical alcoyle renfermant de 1 à 4 atomes de carbone, pour obtenir un produit de formule ($I_D$):

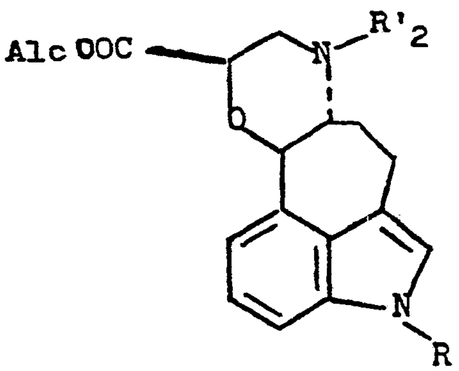

$(I_D)$

dans laquelle Alc, R' et $R'_2$ ont la signification déjà indiquée, que l'on soit l'on isole et, si désiré, salifie, soit l'on soumet à l'action d'un agent d'halogénation pour obtenir un produit de formule ($I_E$):

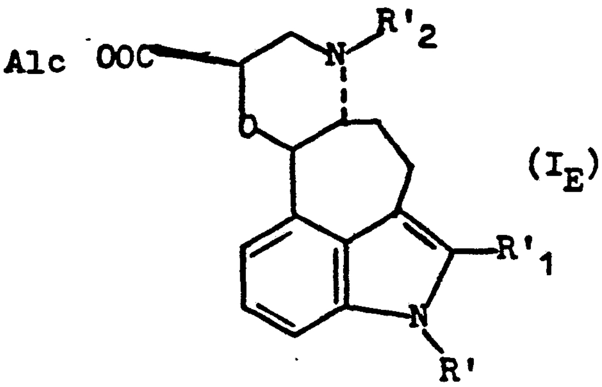

$(I_E)$

dans laquelle Alc, R' et $R'_2$ ont la signification déjà indiquée, et $R'_1$ représente un atome de chlore ou de brome, que l'on isole et salifie si désiré, ou bien l'on soumet ledit produit de formule ($I_C$) à l'action d'un agent d'halogénation pour obtenir un produit de formule ($I_F$):

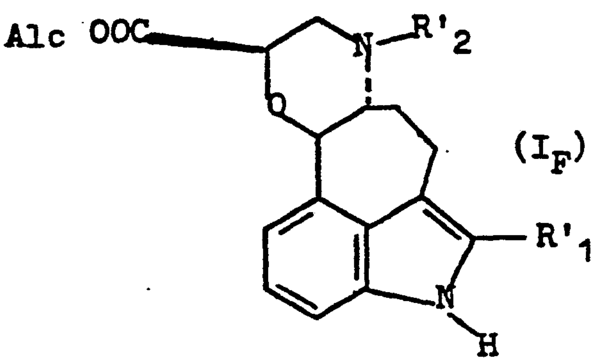

$(I_F)$

dans laquelle Alc, $R'_1$ et $R'_2$ ont la signification déjà indiquée, que l'on isole et si désiré, salifie, soit l'on saponifie ledit produit de formule ($I_A$) pour obtenir in produit de formule ($I_G$):

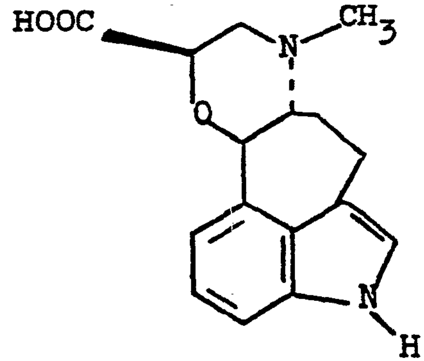

$(I_G)$

que soit l'on isole et, si désiré, salifie, soit l'on fait réagir avec une amine de formule (V):

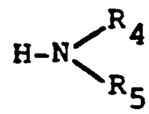

(V)

dans laquelle $R_4$ et $R_5$ ont la signification déjà indiquée, ou avec un peptide, pour obtenir un produit de formule ($I_H$):

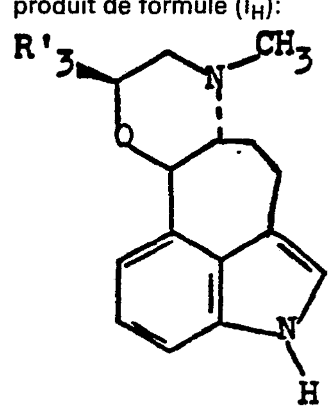

$(I_H)$

dans laquelle $R'_3$ représente un radical carboxy amidifié par l'amine de formule (V) définie ci-dessus, que l'on isole et si désiré, salifie, soit réduit ledit produit de formule ($I_A$) pour obtenir un produit de formule ($I_I$):

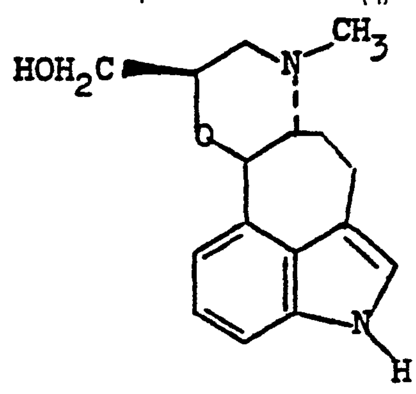

$(I_I)$

que l'on isole et, si désiré, salifie, soit l'on fait réagir avec le chlorure de méthane ou de paratoluène sulfonyle pour obtenir un produit de formule (VI):

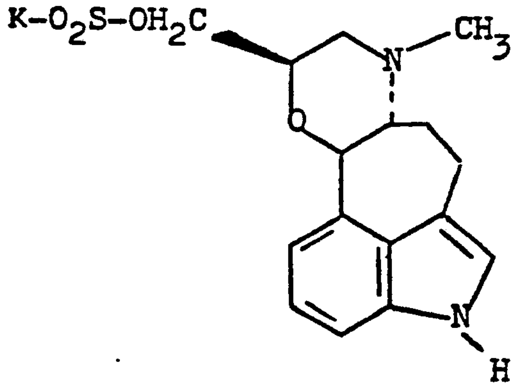

(VI)

dans laquelle K représente un radical méthyle ou p-tolyle, que l'on fait réagir avec un alcoylmercaptan ou avec un cyanure alcalin, pour obtenir un produit de formule ($I_J$):

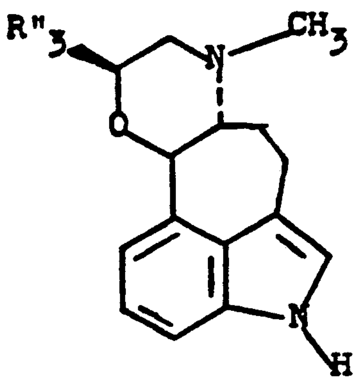

($I_J$)

dans laquelle R"$_3$ représente un radical alcoylthiométhyle ou cyanométhyle que l'on isole et si désiré, salifie, <u>soit</u> l'on oxyde le produit de formule ($I_A$), pour obtenir un produit de formule ($I_K$):

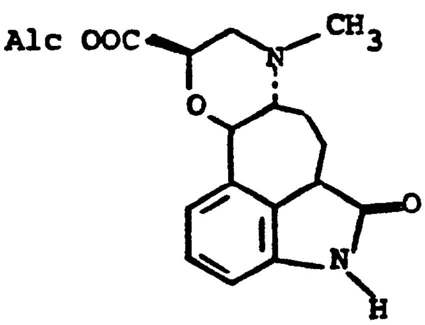

($I_K$)

dans laquelle Alc a la signification déjà indiquée, que ou bien l'on isole et, si désiré, salifie, ou bien l'on réduit pour obtenir un produit de formule ($I_L$):

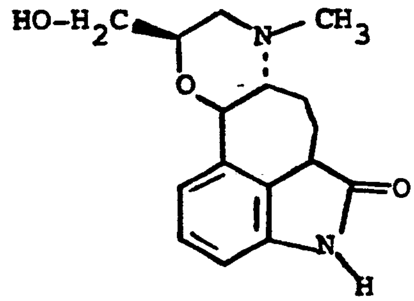

($I_L$)

que l'on isole et si désiré, salifie, puis effectue ensuite si désiré, sur les produits de formule ($I_F$), ($I_G$), ($I_H$), ($I_I$), ($I_J$), ($I_K$) et ($I_L$), une ou plusieurs des réactions déjà indiquées cidessus pour les produits de formule ($I_A$), pour obtenir les produits correspondants entrant dans la formule (I), puis isole, et si désiré, salifie les produits ainsi obtenus.

Dans des conditions préférentielles de mise en oeuvre, le procédé ci-dessus décrit est réalisé comme suit:

- La cyclisation du produit de formule (II) est realisée par actionde la N-chloro diisopropylamine en présence de trisdiméthyl aminophosphine ou d'hexaméthylphosphoramine; on peut également opérer par action du tétrachlorure de carbone, en présence de triphényl phospine ou de trisdiméthyl aminophosphine, puis cyclisation du derivé chloré obtenu par action d'hydrure de sodium dans un solvant tel que le dimethoxyéthane.

- L'épimerisation des produits de formule (III) de structure 8α et 8β est realisée classiquement, de préférence en milieu basique, par exemple par action d'un alcoolate alcalin, notamment l'éthylate de sodium, au reflux pendant 1 à 4 heures.

- L'estérification conduisant au produit de formule($I_A$) est réalisée de préference par action du diazométhane lorsque l'on veut préparer l'ester méthylique.

- La déméthylation du produit de formule ($I_A$)est réalisée de préférence par action du bromure de cyanogéne, suivie d'une réduction telle qu'obtenue par action du zinc dans l'acide acétique.

- L'alcoylation du dérivé de formule ($I_E$)est réalisée de préférence à l'aide d'un halogénure d'alcoyle, notamment un iodure d'alcoyle, en présence d'un agent de condensation tel qu'un carbonate alcalin.

- L'halogénure de formule (IV) peut être un chlorure ou un bromure, mais de préférence un iodure; on le fait réagir après action, notamment dans l'ammoniaque, d'un amidure alcalin, de préférence d'un amidure de sodium, sur le produit de formule ($I_C$).

- L'halogénation des produits de formule ($I_C$ et ($I_D$ peut *52tre realisee par exemple à l'aide de N-chloro succinimide dans le cas de la chloration; elle est réalisée à l'aide de N-bromo succinimide ou de préférence à l'aide du complexe brome de

la pyrrolidone de formule:

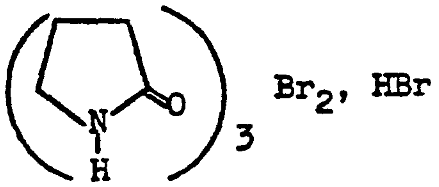

dans le cas de la bromation.

- La saponification du produit de formule ($I_A$) est réalisee de préférence par action d'une base forte telle que la soude 2N; on peut également opérer une hydrolyse en milieu acide, par exemple par action d'un acide minéral tel que l'acide chlorhydrique dilué, dans un solvant tel qu'un alcanol renfermant de 1 à 5 atomes de carbone, notamment l'éthanol.

- La réaction du produit de formule ($I_G$) avec l'amine de formule (V) pour conduire au produit de formule ($I_H$) est réalisée de préférence après activation de la fonction acide carboxylique par formation d'un anhydride mixte par exemple par action d'anhydride trifluoroacétique ou d'un halogénoformiate d'alcoyle tel que le chloroformiate d'isobutyle.

- La réduction des produits de formule ($I_A$) et ($I_K$) est réalisée de preférence par action de borohydrure de sodium, au reflux d'un solvant tel que dioxanne ou le mélange dioxanne-methanol ou dioxanne-éthanol; on peut également utiliser d'autres réducteurs tels l'hydrure d'aluminium-lithium ou le cyano-borohydrure de sodium.

- La réaction du produit de formule ($I_I$) avec le chlorure de méthane ou de paratoluéne sulfonyle est réalisée de préférence dans la pyridine ü température ambiante.

- La réaction du produit de formule (VI) avec l'alcoylmercaptan est réalisée de préférence à température ambiante dans un solvant tel que le diméthylacétamide, en présence d'hydrure de sodium; l'alcoylmercaptan est de préférence le méthylmercaptan. Le cyanure alcalin que l'on fait réagir avec le produit de formule (VI) est de préférence le cyanure de sodium ou de potassium; on opére avantageusement dans un solvant tel que le dimethylformamide.

- L'oxydation du produit de formule ($I_A$) est realisee de preférence ü température ambiante avec le mélange diméthylformamideacide chlorhydrique.

Les dérivés de formule (I) à l'exception des dérivés de formule (I) dans laquelle $R_3$ représente un radical carboxy libre, presentent un caractere basique. On peut avantageusement preparer les sels d'addition des dérivés de formule (I), en faisant réagir, en proportions sensiblement stoechiométriques, un acide mineral ou organique avec ledit derivé de formule (I). Les sels peuvent être préparés sans isoler les bases correspondantes.

Les dérivés de formule (II) peuvent être préparés par réaction d'un produit de formule (VII):

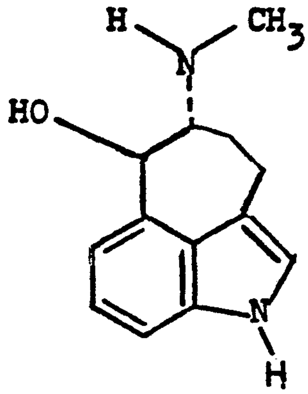
(VII)

avec un glycidate d'alcoyle de formule (VIII):

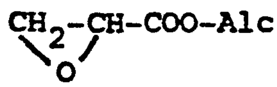
(VIII)

dans laquelle Alc a la signification déjà indiquée, pour obtenir le produit de formule (II) attendu.

Le glycidate d'alcoyle de formule (VIII) est avantageusement le glycidate d'éthyle; on opàre de préférence au reflux d'un alcanol renfermant de 1 à 4 atomes de carbone, de préférence l'alcanol correspondant à l'alcoyle estérifiant, l'acide glycidique.

Les derivés objet de la présente invention, possédent de tràs intéressantes propriétés pharmacologiques; ils sont doués notamment de remarquables propriétés agonistes dopaminergiques, inhibitrices de la sécrétion de prolactine, sérotoninergiques et antihypertensives.

Certaines de ces propriétés sont illustrées plus loin dans la partie expérimentale. Les dérivés objet de la présente invention sont également doués de propriétés antianoxiques.

Ces propriétés justifient l'utilisation des dérivés de la C-homo 9-oxaergoline, ainsi que de leurs sels pharmaceutiquement acceptables, à titre de médicaments.

La présente demande a ainsi également pour objet l'application à titre de médicaments, des dérivés de la C-homo 9-oxaergoline tels que définis par la formule I, ainsi que de leus sels d'addition avec les acides pharmaceutiquement acceptables.

Parmi les médicaments, objet de l'invention, on retient, de préférence, les médicaments caractérisés en ce qu'ils sont constitués par les nouveaux dérivés de la C-homo 9-oxaergoline répondant à la formule (I) dans laquelle a et b forment une double liaison et notamment parmi ceux-ci, ceux dans lesquels R represente un atome d'hydrogène, ainsi que par leurs sels d'addition avec les acides pharmaceutiquement acceptables.

Parmi ces derniers, on retient notamment ceux répondant à la formule I dans laquelle $R_1$ représente un atome d'hydrogène, de chlore, ou de brome, $R_2$ représente un radical alcoyle renfermant de 1 è 4 atomes de carbone, et $R_3$

représente un radical hydroxy méthyl, un radical méthylthiométhyle ou cyanométhyle ou un radical carboxy éventuellement estérifié par un alcool aliphatique linéaire renfermant de 1 è 4 atomes de carbone, ou amidifié par une amine de formule:

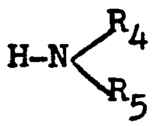

dans laquelle $R_4$ et $R_5$ représentent un radical alcoyle linéaire renfermant de 1 è 4 atomes de carbone, ainsi que leurs sels d'addition avec les acides pharmaceutiquement acceptables.

Parmi ces derniers, on retient tout particulièrement les dérivés dont les noms suivent:

- le /5 RS (5α, 8β, 10β)/ 6-méthyl C-homo 9-oxaergoline-8-carboxylate d'éthyle
- le /5 RS (5α, 8β, 10β)/ 6-méthyl C-homo 9-oxaergoline-8-carboxylate de méthyle et
- le /5 RS (5α, 8β, 10β)/ 6-méthyl C-homo 9-oxaergoline-8-méthanol,

ainsi que leurs sels d'addition avec les acides pharmaceutiquement acceptables.

Les médicaments selon l'invention trouvent leur emploi, dans le traitement des syndromes neurologiques d'origine extrapyramidale, par exemple dans le traitement de la maladie de Parkinson, et dans le traitement des syndromes parkinsonniens post-encéphalitiques; ils peuvent également être utilisés dans le traitement de l'hypersécrétion de prolactine par l'antehypophyse, par exemple dans le traitement de l'hypogonadisme de la femme ou de l'homme. Ils peuvent aussi être utilises dans le traitement de l'hypertension artérielle essentielle, de l'hypertension de la cinquantaine, de la menopause, du diabétique, de l'obèse et du pléthorique, ainsi que dans le traitement de l'hypertension artérielle du sujet âge ou atteint d'arteriosclérose et dans le traitement de l'hypertension d'origine rénale. Ils peuvent aussi être utilisés dans le traitement de la senescence cérebrale ou des manifestations liées à une hypoxie cérébrale.

La dose usuelle, variable selon le produit utilisé, le sujet traité et l'affection en cause, peut être par exemple de 5 mg à 200 mg par jour, par voie orale, chez l'homme, du produit de l'exemple 2.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables, peuvent être incorporés dans des compositions pharmaceutiques destinées à la voie digestive ou parentérale.

Ces compositions pharmaceutiques peuvent être solides ou liquides et se présenter sous les formes pharmaceutiques couramment utilisées en médecine humaine, comme par exemple les comprimés simples ou dragéifiés, les gélules, les granulés, les suppositoires ou les préparations injectables; ces formes peuvent être préparées selon les méthodes usuelles; le ou les principe actifs peuvent y être incorporés ü des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants ou les conservateurs.

L'invention s'étend en outre aux produits industriels nouveaux utiles notamment pour la préparation des dérivés répondant à la formule I, à savoir les produits de formule II:

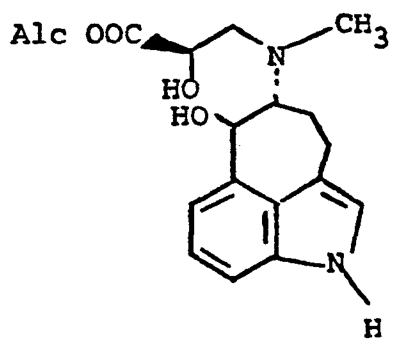

(II)

dans laquelle Alc a la signification dejà indiquée

Il va être donné maintenant à titre non limitatif des exemples de mise en oeuvre de l'invention.

**EXEMPLE No 1: /5 RS (5α, 8β, 10β 6-méthyl C-homo 9-oxaergoline-8-carboxylate d'éthyle**

On dissout sous agitation et atmosphère inerte 13,28 g de 3-//(5 RS trans) 6-hydroxy 3,4,5,6-tétrahydro 1H-cyclohept (c,d) indol-5-yl/méthyl amino/2-hydroxy propanoate d'éthyle et 10,8 g de N-chlorodiisopropylamine dans 100 cm3 de diméthylformamide, refroidit à - 40°C, ajoute goutte à goutte en 15 mn 17,2 cm3 de trisdiméthyl amino phosphine, agite à - 40° C pendant 15 mn, puis pendant 2 heures sans réfrigeration, ajoute de l'eau, extrait au chlorure de méthylène, sèche, amène à sec sous pression réduite, purifie par chromatographie sur silice (éluant: chlorure de méthylèneméthanol 97-3) et obtient en 3 fractions le produit attendu, soit:

700 mg d'isomère 8 α(F = 148° C).
3,3 g de mélange d'isomère 8α et 8β
6 g d'isomère 8β(F=110° C).

Le 3-//(5 RS trans) 6-hydroxy 3,4,5,6-tétrahydro IHcyclohept (c,d) indol-5-yl/ méthyl amino/ 2-hydroxy propanoate d'éthyle de départ peut être préparé comme suit:

On agite au reflux, pendant 3 heures, sous atmosphère inerte, 32,7 g de RS-trans 5-méthylamino 3,4,5,6-tétrahydro IH-cyclohept (c,d) indol-6-ol décrit dans le brevet français n°2476649, et 27 g de glycidate d'éthyle, dans 600 cm3 d'éthanol, évapore à sec, reprend au chlorure de méthylène et extrait par 5 fois 100 cm3 d'acide chlorhydrique 2N. On refroidit la fraction acide, alcalinise par de la lessive de soude, extrait au chlorure de méthylène à 5 % de méthanol, lave, sèche, concentre ü sec sous pression réduite, reprend par un mélange d'éther et de chlorure de méthylène, laisse cristalliser, essore, lave à l'éther, sèche et obtient 26 g du produit attendu F=130° C.

**EXEMPLE No 2: /5 RS (5α, 8β, 10β)/ 6-méthyl C-homo 9oxaergoline-8-méthanol et son chlorhydrate**

On dissout, sous agitation et atmosphère inerte, 5,5 g d'isomère 8β obtenu ü l'exemple 1 dans 100 cm3 d'éthanol et 100 cm3 de dioxanne, ajoute 5,5 g de borohydrure de sodium, porte pendant 1 h 30 au reflux, refroidit, ajoute de l'eau extrait au chlorure de méthylène à 10 % de méthanol, lave à l'eau, sèche, amène à sec sous pression réduite, empçte dans l'éther, essore et obtient 4,10 g du produit attendu F≃255 à 260°C.

**Formation du chlorhydrate**

On chauffe à 70°C dans 140 cm3 de méthanol, 2,85 g de la base obtenue ci-dessus, ajoute 5 cm3 d'éther chlorhydrique, filtre, laisse cristalliser, essore, lave, sèche et obtient 2,64 g du produit attendu F>250°C avec décomposition.

**Analyse**

pour $C_{16}$ $H_{21}$ Cl $N_2$ $0_2$≃308,80
calculé C% 62,23 H%6,85 N% 9,07 C1% 11,48
trouvé 62,4 6,9 9,1 11,6

**EXEMPLE No 3: /5 RS (5α, 8β, 10β)/ 6-méthyl C-homo 9oxaergoline-8-carboxylate de méthyle, et son chlorhydrate. Stade A: Epimérisation du mélange d'isomè res 8α et 8β du /5 RS (5α, 8β 10β)/ 6-méthyl C-homo 9-oxaergoline-8carboxylate d'éthyle**

On dissout sous atmosphère inerte 960 mg de sodium dans 50 cm3 d'éthanol, ajoute 3,5 g du mélange d'somères de l'exemple no 1 et 50 cm3 d'éthanol, porte au reflux pendant 4 heures, amène à sec sous pression réduite, ajoute de l'eau et neutralise à froid à l'aide d'acide chlorhydrique concentré; on obtient ainsi l'acide libre 8β correspondant.

**Stade B: Estérification**

On reprend le produit ci-dessus par 100 cm3 de chlorure de méthylène et 20 cm3 de méthanol, ajoute goutte à goutte, à 0° C, 100 cm3 de diazométhane en solution dans le chlorure de méthylène, glace pendant 48 heures, élimine l'excès de diazométhane à l'aide d'acide acétique, lave avec une solution d'hydroxyde de sodium 2N, puis à l'eau, sèche, et améne à sec sous pression réduite. On purifie le résidu par chromatographie sur silice (Eluant: benzène-acétate d'éthyle 50-50) et obtient 1,96 g du produit attendu F≃80° C.

**Formation du chlorhydrate:**

On dissout à chaud 1,33 g du produit ci-dessus dans 100 cm3 d'éther éthylique, filtre, ajoute 4 cm3 d'éther chlorhydrique, laisse cristalliser, essore, lave, sèche, et obtient 1,25 g du chlorhydrate attendu F 250° C.

**Analyse:**

pour $C_{17}$ $H_{20}$ $N_2$ $0_3$ HCl = 336,81
calculé C% 60,62 H% 6,28 N% 8,32 C1% 1o,53
trouvé 60,7 6,3 8,3 10,6

**Exemple 4: /5RS(5α,8β, 10β)/ 6-méthyl C-homo 9-oxaergoline 8-methylthiométhyle et son chlorhydrate.**

**Stade A: Ester paratoluè ne sulfonique de /5RS(5α,8β,10β)/ 6méthyl C-homo 9-oxaergoline 8-méthanol.**

On introduit 8,4 g de /5RS(5α, 8β, 10β)/ 6-méthyl C-homo 9-oxaergoline 8-méthanol obtenu à l'exemple 2 dans 150 cm3 de pyridine. On ajoute, goutte à goutte, 11,7 g de chlorure de tosyle dans 75 cm3 de pyridine et agite pendant 16 heures à température ambiante. On verse le mélange réactionnel dans l'eau, extrait au chlorure de methylène, lave avec une solution aqueuse de bicarbonate de sodium, sèche et évapore à sec sous pression réduite. On reprend le résidu par de l'éther, essore, lave à l'éther et obtient 10,7 g de produit attendu. F = 152°C.

**Stade B: /5RS(5α,8β,10β)/ 6-méthyl C-homo 9-oxaergoline 8-méthylthiomethyle et son chlorhydrate.**

A 50 cm3 de méthylmercaptan, on ajoute 100 cm3 de diméthylacétamide anhydre, refroidit à 0°C pour ajouter par petites fractions, 10 g d'hydrure de sodium à 50% dans l'huile de vaseline. A température ambiante, on ajoute, goutte à goutte, au mélange réactionnel, 5 g de l'ester paratoluène sulfonique obtenu au stade A dans 50 cm3 de diméthylacétamide. On agite 2 heures à température ambiante, verse dans un mélange eau et glace et extrait au chlorure de methylène. Les phases organiques sont lavées ü l'eau, sèchées et concentrees a sec sous pression réduite. On chromatographie sur silice le résidu obtenu, en éluant par un système benzène-acétate d'éthyle (7/3). On obtient 2,85 g de produit attendu. F = 158°C.

On dissout 2,8 g de ce dernier dans 100 cm3 de méthanol, filtre, ajoute 5 cm3 de methanol chlorhydrique, laisse 1 heure à 0°+5°C. On essore le chlorhydrate que l'on recristallise dans le méthanol. On isole 2,1 g de chlorhydrate du produit attendu. F = 245°C.

**Exemple 5: /5RS(5α,8β,10β)/ 6-methyl C-homo 9-oxaergoline 8acetonitrile et son chlorhydrate.**

A 5,5 g d'ester paratoluène sulfonique préparé au stade A de l'exemple 4 dans 55 cm3 de diméthylformamide, on ajoute 12,75 g de cyanure de sodium dans 25 cm3 d'eau et 50 cm3 de diméthylformamide. On agite 16 heures à 60°C, refroidit, verse dans l'eau, extrait au chlorure de méthylène, lave à 1,eau, sèche et concentre à sec sous pression réduite. On empâte le résidu obtenu à l'éther, essore et obtient 3,3 g de produit attendu. E = 224°C.

On le dissout dans 150 cm3 de chlorure de méthylène, filtre, ajoute 5 cm3 de methanol chlorhydrique, refroidit et essore le chlorhydrate brut que l'on recristallise dans le méthanol pour obtenir 3 g de chlorhydrate du produit attendu. F > 250°C.

**Exemple 6: /5RS(5α; 8β,10β)/ 6-méthyl C-homo 9-oxaergoline 8N,N-diéthylcarboxamide et son chlorhydrate.**

On refroidit à ˉ 10°C 3,1 g d'acide /5RS(5α,8β,10β)/ 6-méthyl C-homo 9-oxaergoline 8-carboxylique obtenu à l'exemple 3, 80 cm3 de diméthylformamide anhydre et 80 cm3 de dioxanne anhydre. On ajoute le mélange: 3,2 cm3 de tributylamine et 1,7 cm3 de chloroformiate d'isobutyle et agite 1 heure à temperature ambiante. On verse le mélange réactionnel en une seule fois dans 50 cm3 de dioxanne et 8 cm3 de diéthylamine et agite 1 heure à température ambiante. On verse dans l'eau, extrait au chlorure de méthylène, lave à l'eau, sèche et concentre à sec sous pression réduite. On reprend le résidu à l'éther et obtient 2,33 g de produit. F = 178-180°C.

On récupère 0,4 g de produit des liqueurs mères après passage sur silice (éluant: $CH_2Cl_2$-MeOH 95/5) F = 180°C.

On dissout 2,7 g de base libre dans 50 cm3 de méthanol, filtre, ajoute 3 cm3 d'éther chlorhydrique (3 ou 4N) et laisse cristalliser à température ambiante. Après essorage, on obtient le chlorhydrate que l'on recristallise dans du méthanol. On isole 2,35 g de chlorhydrate du produit attendu. F≃245-250°C.

**Exemple 7: /5RS(5α,8β, 10β)/ 2-bromo 6-méthyl C-homo 9-oxaerqoline 8-carboxylate d'éthyle et son chlorhydrate.**

**Stade A: /5RS(5α,8β,10β)/ 2-bromo 6-méthyl C-homo 9-oxaergoline 8-carboxylate d'éthyle.**

On dissout 18,72 g d'hydrotribromure de pyrrolidone dans 2,4 litres de dioxanne à température ambiante et introduit en 5 minutes 9 g de /5RS(5α,8β,10β)/ 6-methyl C-homo 9-oxaergoline 8-carboxylate d'ethyle obtenu à l'exemple 1. On evapore le dioxanne et reprend le résidu par une solution saturée de carbonate de sodium et de l'acétate d'éthyle, agite, décante, lave la phase organique à l'eau saturée de chlorure de sodium, sèche et évapore à sec sous pression réduite. Le résidu est chromatographié sur silice et élué par un mélange cyclohexane-acétate d'éthyle (5/5). on obtient 6,78 g de produit attendu. F = 160°C.

**Stade B: /5RS(5α,8β,10β)/ 2-bromo 6-méthyl C-homo 9-oxaergoline 8-carboxylate d'éthyle et son chlorhydrate.**

Dans 90 cm3 de méthanol et 45 cm3 de dioxanne, on dissout à température ambiante 4,5 g du produit obtenu ci-dessus, ajoute 4,5 g de borohydrure de sodium et porte au reflux pendant 1 heure. Après refroidissement de la suspension à température ambiante, on ajoute 100 cm3 d'eau, agite 30 minutes, filtre, rince à l'eau et obtient 3,5 g de produit attendu. F > 260°C.

On met en suspension 3 g de ce dernier dans 40 cm3 de methanol, refroidit à 0+5°C et ajoute goutte à goutte en agitant de l'éther chlorhydrique jusqu'à pH 1. On agite 2 heures à température ambiante, filtre, rince à l'éther et obtient 3,4 g de chlorhydrate attendu. F = 255°C.

**Exemple 8: /5RS(5α,8β,10β)/ 2,3-dihydro 2-oxo 6-méthyl C-homo 9-oxaerqoline 8-méthanol et son chlorhydrate.**

**Stade A: /5RS(5α,8β,10β)/ 2,3-dihydro 2-oxo 6-méthyl C-homo 9oxaergoline 8-carboxylate d'éthyle.**

Dans 45 cm3 de diméthylsulfoxyde, on introduit 90 cm3 d'acide chlorhydrique concentré et agite pour faire baisser la température à 20°C. On ajoute rapidement 9 g de /5RS(5α,8β,10β)/ 6-méthyl C-homo 9-oxaergoline 8-carboxylate d'éthyle obtenu à l'exemple 1, agite 30 minutes à température ambiante, refroidit à 0+5°C et neutralise avec de la triéthylamine, puis essore. On dissout les cristaux obtenus dans du chlorure de méthylène à 10% de méthanol, sèche puis concentre à sec et obtient 3,1 g de produit attendu après recristallisation dans l'éther. F = 264°C.

**Stade B: /5RS(5α,8β,10β)/ 2,3-dihydro 2-oxo 6-méthyl C-homo 9oxaergoline 8-méthanol et son chlorhydrate.**

Dans 30 cm3 d'éthanol et 10 cm3 de dioxanne, on dissout à température ambiante 3,1 g de produit obtenu ci-dessus, ajoute 3,1 g de borohydrure par petites portions, et porte au reflux pendant 2 heures. Après refroidissement à température ambiante, on verse le mélange réactionnel sur de la glace, sature de carbonate de potassium, extrait au chlorure de méthylène, lave à l'eau saturée de chlorure de sodium, sèche et concentre à sec On obtient 1,96 g de produit attendu. F = 220°C. On met en suspension 1,85 g de ce dernier dans 10 cm3 de méthanol, refroidit à 0°C et ajoute de l'éther chlorhydrique jusqu'à pH 1 puis agite 30 minutes à température ambiante. On obtient 1,74 g de chlorhydrate attendu. F = 260°C.

**EXEMPLE No 9**

On a préparé des comprimés répondant à la formule:

- chlorhydrate de /5 RS (5α, 8β, 10β)/ 6-méthyl C-homo 9-oxaergoline-8-méthanol........................... 10 mg;
- excipient q. s. pour un comprimé terminé à... 100 mg. (Détail de l'excipient: lactose, amidon, talc, stéarate de magnésium).

**Etude pharmacologique:**

1°) Comportement de rotation après lésion unilatérale du faisceau nigrostriatal par la 6-hydroxydopamine.

**Technique**

La lésion est effectuée chez des rats mçles de 220 g environ par injection unilatérale dans le faisceau dopaminergique nigrostrié, de 8µg de 6-hydroxydopamine en solution à 2 µg/µl (U. Ungerstedt, Acta Physiol. Scand. 1971, 82, suppl. 367, 69-93).

Chez de tels animaux, les agonistes dopaminergiques directs, tels que l'apomorphine, administrés par voie générale, entrainent un comportement de rotation dans la direction contralatérale au côté lésé.

Le composé étudié est administré au moins 5 semaines après la lésion. Les animaux sont placés dans un rotomètre automatisé qui permet de compter le nombre de rotations effectuées par chaque animal dans les deux sens.

Le produit de l'exemple 2 administré par voie intrapéritonéale provoque des rotations contralatérales dès la dose de 0,5 mg/kg.

2°) Détermination de l'activité hypotensive.

L'activité hypotensive a été étudiée sur des rats mç1es de souche WISTAR pesant 300 g environ et anesthésiés au nembutal (50 mg/kg par voie intraveineuse).

Le produit testé a éte administré par voie intraveineuse dans la veine jugulaire.

La pression artérielle carotidienne a été mesurée avant et après administration du produit teste.

Le tableau ci-après indique les variations exprimées en pourcentage de la pression artérielle après administration du produit testé, par rapport à la pression artérielle témoin initiale.

| Produits de l'exemple | Dose mg/kg | Variation % de la pression artérielle | | | |
|---|---|---|---|---|---|
| | | 1 mn après administration | 5 mn | 10 mn | 30 mn |
| 2 | 10 | - 44 | - 40 | - 37 | - 31 |
| | 1 | - 29 | - 25 | - 20 | - 11 |

3°) Etude de la toxicité aigüe.

On a évalué les doses létales $DL_0$ des différents composés testés après administration par voie intrapéritonéale chez la souris.

On appelle $DL_0$ la dose maximale ne provoquant aucune mortalité en 8 jours.

Les résultats obtenus sont les suivants:

| Produits de l'exemple | $DL_0$ en mg/kg |
|---|---|
| 2 | 100 |

**Revendications** pour les Etats contractants BE CH DE FR GB IT LI LU NL SE

1°) Dérivés de la C-homo 9-oxaergoline, ainsi que leurs sels d'addition avec les acides minéraux ou organiques caractérisés en ce qu'ils répondent à la formule générale (I):

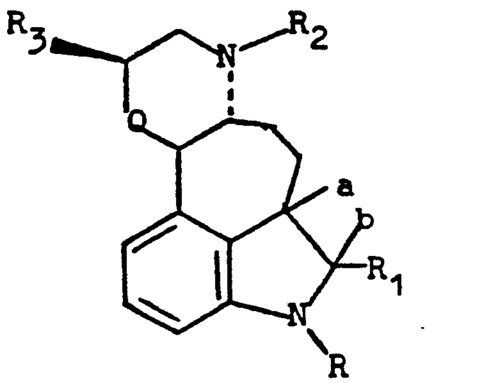

(I)

dans laquelle R représente un atome d'hydrogène ou un radical alcoyle renfermant de 1 à 4 atomes de carbone, $R_1$ représente un atome d'hydrogène, de chlore ou de brome ou forme avec b un radical oxo, a forme avec b une double liaison ou a représente un atome d'hydrogène quand b forme avec $R_1$ un radical oxo, b forme avec a une double liaison ou forme avec $R_1$ un radical oxo, $R_2$ représente un atome d'hydrogène, un radical alcoyle renfermant de 1 à 4 ütomes de carbone, aralcoyle renfermant de 7 à 12 atomes de carbone, ou cycloalcoylalcoyle renfermant de 4 à 7 atomes de carbone, $R_3$ représente un radical hydroxyméthyle, alcoylthiométhyle, cyanométhyle ou carboxy éventuellement esterifié par un alcool aliphatique renfermant de 1 à 5 atomes de carbone, ou amidifié par une amine de formule

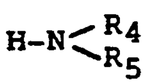

dans laquelle $R_4$ représente un atome d'hydrogène ou un radical alcoyle renfermant de 1 à 4 atomes de carbone, et $R_5$ représente un radical alcoyle renfermant de 1 à 4 atomes de carbone ou $R_4$ et $R_5$ forment ensemble avec l'atome d'azote un hétérocycle saturé renfermant éventuellement un autre hétéroatome, ce dernier pouvant être substituél par un radical alcoyle renfermant de 1 à 4 atomes de carbone quand l'hétérocycle renferme un dutre atome däazote.

2°) Dérivés de la C-homo 9-oxaergoline, ainsi que leurs sels d'addition avec les acides minéraux ou organiques, caractérisés en ce qu'ils répondent à la formule (I) de la revendication 1, dans laquelle a et b forment une double liaison.

3°) Dérivés de la C-homo 9-oxaergoline, ainsi que leurs sels d'addition avec les acides minéraux ou organiques, caractérisés en ce qu'ils répondent à la formule (I) de la revendication 2, dans laquelle R représente un atome d'hydrogene.

4°) Dérivés de la C-homo 9-oxaergoline selon la revendication 3, ainsi que leurs sels d'addition avec les acides minéraux ou organiques, caractérisés en ce que $R_1$ représente un atome d'hydrogène, de chlore ou de brome, $R_2$ représente un radical alcoyle renfermant de 1 à 4 atomes de carbone et $R_3$ représente un radical hydroxyméthyle, méthylthiométhyle ou cyanométhyle, un radical carboxy éventuellement estérifié par un alcool aliphatique linéaire renfermant de 1 è 4 atomes de carbone ou amidifié par une amine de formule

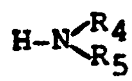

dans laquelle

$R_4$ et $R_5$ représentent un radical alcoyle linéaire renfermant de 1 à 4 atomes de carbone.

5°) L'un quelconque des produits selon la revendication 1 dont les noms suivent:

- le /5 R8 (5α, 8β, 10β)/ 6-méthyl C-homo 9-oxaergoline-8-carboxylate d'éthyle
- le /5 R8 (5α, 8β, 10β)/ 6-méthyl C-homo 9-oxaergoline-8-carboxylate de méthyle et
- le /5 R8 (5α, 8β, 10β)/ 6-méthyl C-homo 9-oxaergoline-8-méthanol,

ainsi que leurs sels d'addition avec les acides minéraux ou organiques.

6°) procédé de préparation des dérivés de la C-homo 9-oxaergo-line tels que définis par la formule (I) de la revendication 1, ainsi que de leurs sels, caractérisé en ce que l'on cyclise un produit de formule (II):

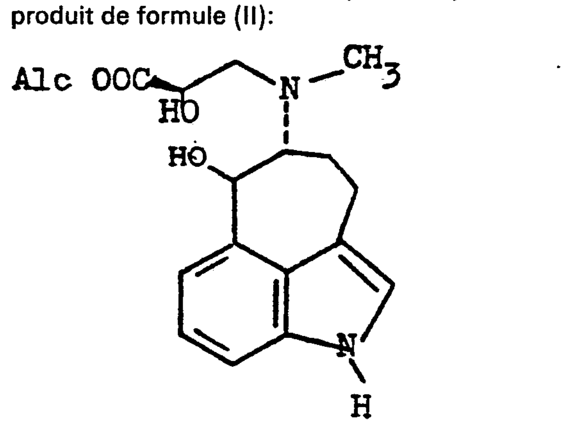

(I:

dans laquelle Alc représente un radical alcoyle renfermant de 1 à 4 atomes de carbone, pour obtenir un produit de formule (III);

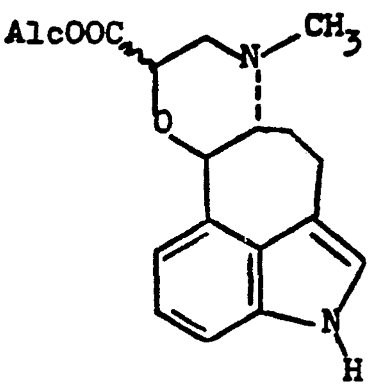

(III)

dans laquelle Alc a la signification déjà indiquée, et le trait ondulé signifie que le substituant est en position 8α ou 8β, dont, soit l'on isole l'isomère 8β, soit l'on épime rise l'isomère 8α puis estérifie l'acide ainsi obtenu dans lequel le substituant en 8 est en position 8β, pour obtenir le produit de formule ($I_A$):

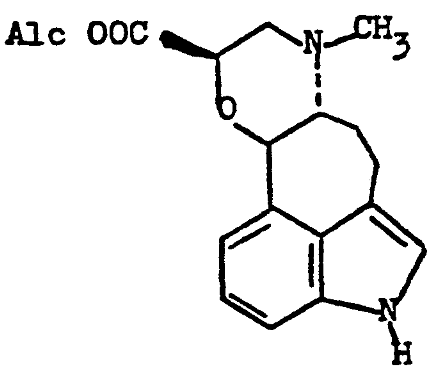

($I_A$)

dans laquelle Alc a la signification déjà indiquée, que

- soit l'on isole et si désiré, salifie,
- soit l'on déméthyle pour obtenir le produit de formule ($I_B$):

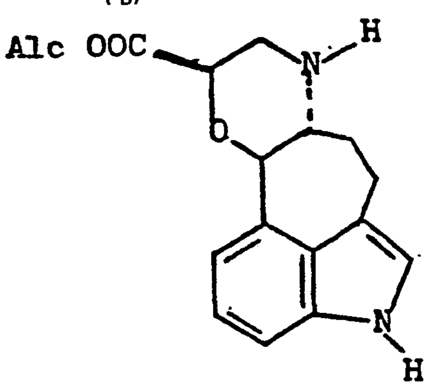

($I_B$)

dans laquelle Alc a la signification déjà indiquée, que soit l'on isole et si désiré, salifie, soit l'on fait réagir avec un agent d'alcoylation pour obtenir un produit de formule ($I_C$):

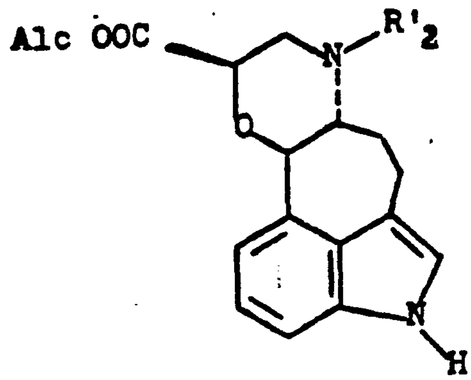

($I_C$)

dans laquelle $R'_2$ a la signification de $R_2$ déjà indiquée à l'exception de l'hydrogène, que ou bien l'on isole, et si désiré, salifie ou bien l'on fait réagir avec un amidure alcalin, puis avec un halogénure d'alcoyle de formule (IV):

Hal-R' (IV)

dans laquelle Hal représente un atome de chlore, de brome ou d'iode et R' représente un radical alcoyle renfermant de 1 à 4 atomes de carbone, pour obtenir un produit de formule ($I_D$):

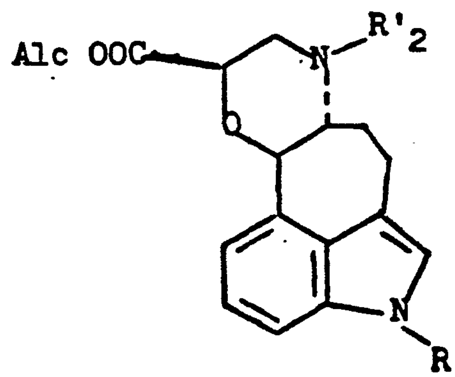

($I_D$)

dans laquelle Alc, R' et $R'_2$ ont la signification déjà indiquée, que soit l'on isole et, si désiré, salifie, soit l'on soumet à l'action d'un agent d'halogénation pour obtenir un produit de formule ($I_E$):

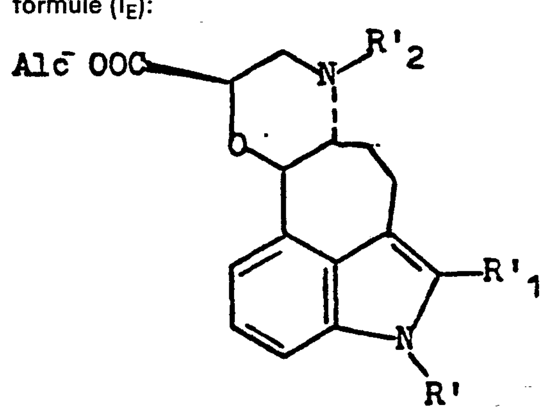

($I_E$)

dans laquelle Alc, R' et $R'_2$ ont la signification déjà indiquée, et $R'_1$ représente un atome de chlore ou de brome que l'on isole et salifie si désiré, ou bien l'on soumet ledit produit de formule ($I_C$) à l'action d'un agent d'halogénation pour obtenir un produit de formule ($I_F$):

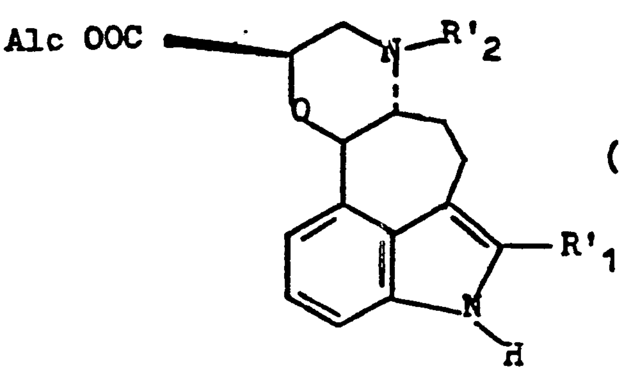

$(I_F)$

dans laquelle Alc, $R'_1$ et $R'_2$ ont la signification déjà indiquee, que l'on isole et si désiré, salifie,

soit l'on saponifie ledit produit de formule $(I_A)$ pour obtenir un produit de formule$(I_G)$:

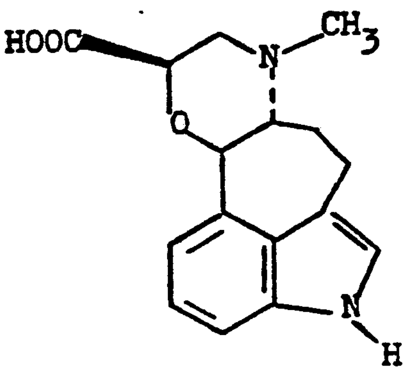

$(I_G)$

que soit l'on isole et, si désiré, salifie, soit l'on fait réagir avec une amine de formule (V):

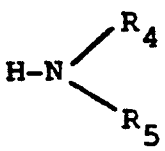

(V)

dans laquelle $R_4$ et $R_5$ ont la signification déjà indiquée, ou avec un peptide, pour obtenir un produit de formule $(I_H)$:

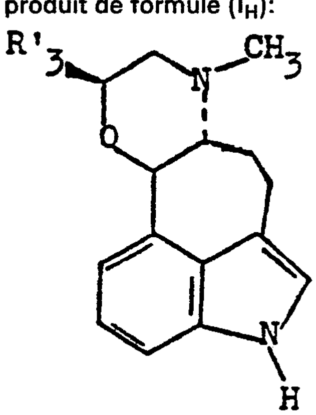

$(I_H)$

dans laquelle $R'_3$ représente un radical carboxy amidifié par l'amine de formule (V) définie ci-dessus, que l'on isole et si désiré, salifie,

soit réduit ledit produit de formule $(I_A)$ pour obtenir un produit de formule $(I_I)$:

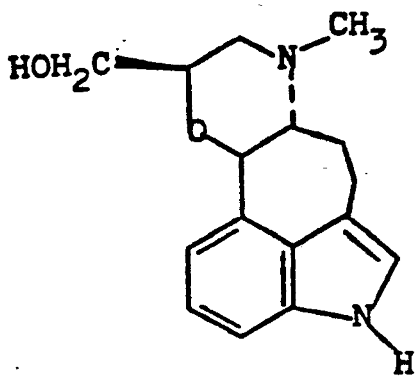

$(I_I)$

que soit l'on isole et, si désiré, salifie, soit l'on fait réagir avec le chlorure de méthane ou de paratoluène sulfonyle pour obtenir un produit de formule (VI):

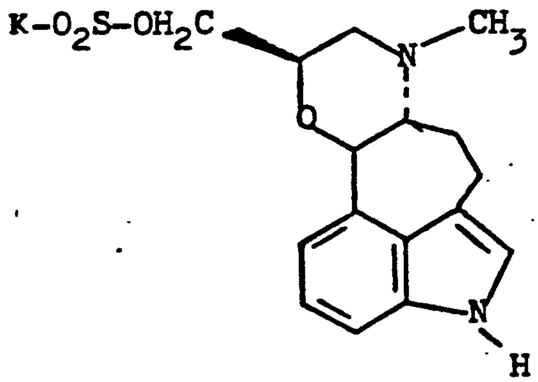

(VI)

dans laquelle K représente un radical méthyle ou p-tolyle, que l'on fait réagir avec un alcoylmercaptan ou avec un cyanure alcalin, pour obtenir un produit de formule $(I_J)$:

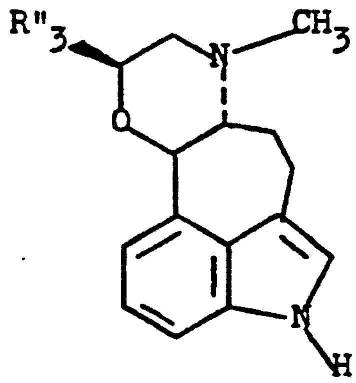

$(I_J)$

dans laquelle $R''_3$ représente un radical alcoylthiométhyle ou cyanométhyle que l'on isole et si désiré, salifie,

soit l'on oxyde le produit de formule $(I_A)$, pour obtenir un produit de formule $(I_K)$:

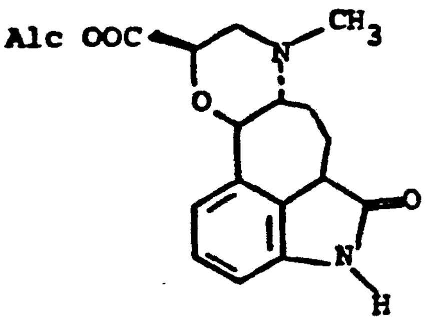

($I_K$)

dans laquelle Alc a la signification déjà indiquée, que ou bien l'on isole et, si désiré, salifie, ou bien l'on réduit pour obtenir un produit de formule ($I_L$):

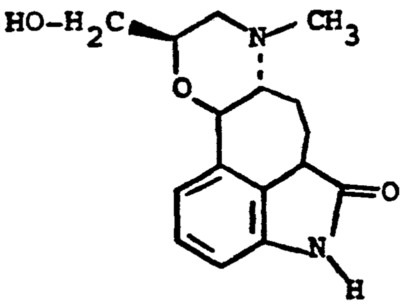

($I_L$)

que l'on isole et si désiré salifie, puis effectue ensuite si désiré, sur les produits de formules ($I_F$), ($I_G$), ($I_H$), ($I_I$), ($I_J$), ($I_K$) et ($I_L$), une ou plusieurs des réactions déjà indiquées ci-dessus pour les produits de formule ($I_A$), pour obtenir les produits correspondants entrant dans la formule (I), puis isole, et si désiré, salifie les produits ainsi obtenus.

7°) Médicaments, caractérisés en ce qu'ils sont constitués par les nouveaux dérivés de la C-homo 9-oxaergoline, tels que définis par la formule (I) de la revendication 1, ainsi que par leurs sels d'addition avec les acides pharmaceutiquement acceptables.

8°) Médicaments, caractérisés en ce qu'ils sont constitués par les nouveaux dérivés de la C-homo 9-oxaergoline, tels que définis à la revendication 2, ainsi que par leurs sels d'addition avec les acides pharmaceutiquement acceptables.

9°) Médicaments, caractérisés en ce qu'ils sont constitués par les nouveaux dérivés de la C-homo 9-oxaergoline, tels que définis à la revendication 3 ou 4, ainsi que par leurs sels d'addition avec les acides pharmaceutiquement acceptables.

10°) Médicaments, caractérisés en ce qu'ils sont constitués par les dérivés de la C-homo 9-oxaergoline, tels que définis à la revendication 5, ainsi que par leurs sels d'addition avec les acides pharmaceutiquement acceptables.

11°) Compositions pharmaceutiques, caractérisées en ce qu'elles renferment, à titre de principe actif, l'un au moins des médicaments, tels que définis à l'une des revendications 7 à 10.

12°) A titre de produits industriels nouveaux nécessaires pour la préparation des dérivés répondant à la formule (I) de la revendication 1:

- les derivés de formule (II):

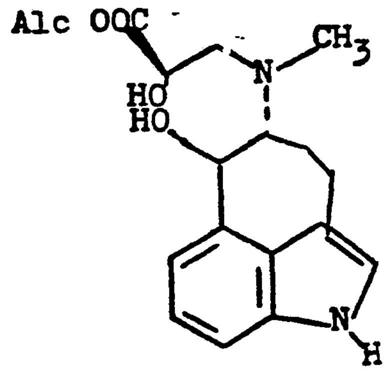

(II)

dans laquelle Alc a la signification déjà indiquée dans la revendication 6.

**Revendications** pour l'Etat contractant AT

1.- Procédé de préparation de nouveaux dérivés de la C-homo 9-oxaergoline, ainsi que de leurs sels d'addition avec les acides minéraux ou organiques, répondant à la formule générale (I):

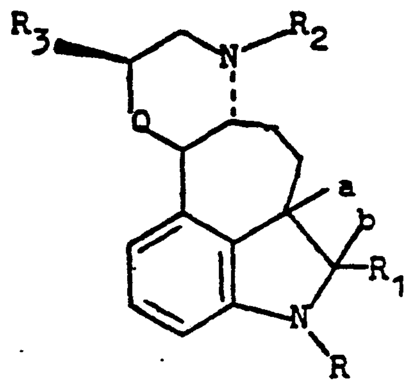

(I)

dans laquelle R représente un atome d'hydrogène ou un radical alcoyle renfermant de 1 à 4 atomes de carbone, $R_1$ représente un atome d'hydrogène, de chlore ou de brome ou forme avec b un radical oxo, a forme avec b une double liaison ou a représente un atome d'hydrogène quand b forme avec $R_1$ un radical oxo, b forme avec a une double liaison ou forme avec $R_1$ un radical oxo, $R_2$ représente un atome d'hydrogène, un radical alcoyle renfermant de 1 à 4 atomes de carbone, aralcoyle renfermant de 7 à 12 atomes de carbone, ou cycloalcoylalcoyle renfermant de 4 à 7 atomes de carbone, $R_3$ représente un radical hydroxyméthyle, alcoylthiométhyle, cyanométhyle ou carboxy éventuellement esterifié par un alcool aliphatique renfermant de 1 à 5 atomes de carbone, ou amidifié par une amine de formule

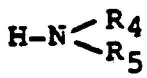

dans laquelle

$R_4$ représente un atome d'hydrogène ou un radical alcoyle renfermant de 1 à 4 atomes de carbone, et $R_5$ représente un radical alcoyle renfermant de 1 à 4 atomes de carbone ou $R_4$ et $R_5$ forment ensemble avec l'atome d'azote un hétérocycle saturé renfermant éventuellement un autre hétéroatome, cedermier pouvant être substitué par un radical alcoyle renfermant de 1 à 4 atomes de carbone quard l'hétérocycle renferme un autre atome d'atote, caracterisé en ce que l'on cyclise un produit de formule (II):

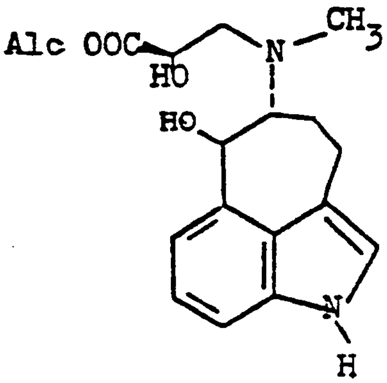

(II)

dans laquelle Alc représente un radical alcoyle renfermant de 1 à 4 atomes de carbone, pour obtenir un produit de formule (III):

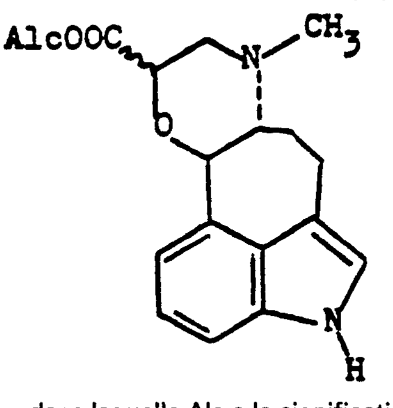

(III)

dans laquelle Alc a la signification déjà indiquée, et le trait ondulé signifie que le substituant est en position 8α ou 8β, dont, soit l'on isole l'isomère 8β, soit l'on épime rise l'isomère 8α puis estérifie l'acide ainsi obtenu dans iequel le substituant en 8 est en position 8β, pour obtenir le produit de formule($I_A$):

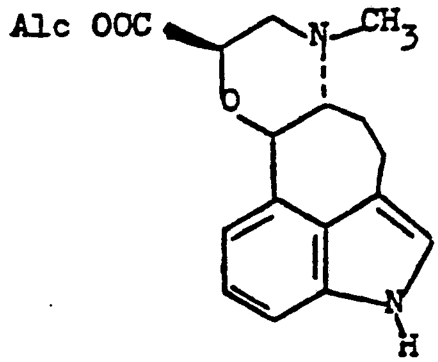

($I_A$)

dans laquelle Alc a la signification déjà indiquée, que

- soit l'on isole et si désiré, salifie,
- soit l'on déméthyle pour obtenir le produit de formule ($I_B$):

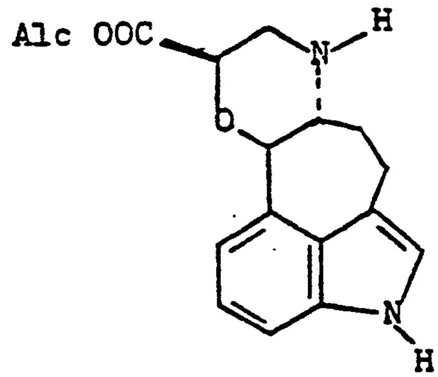

($I_B$)

dans laquelle Alc a la signification déjà indiquée, que soit l'on isole et si désiré, salifie, soit l'on fait réagir avec un agent d'alcovlation pour obtenir un produit de formule ($I_C$).

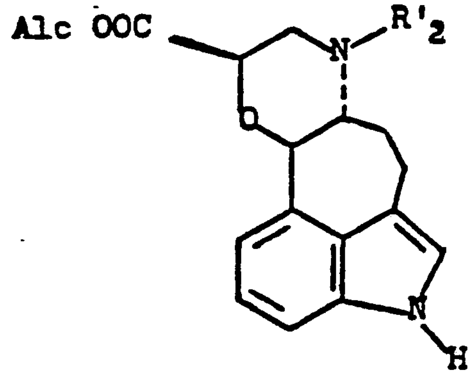

($I_C$)

dans laquelle $R'_2$ a la signification de $R_2$ déjà indiquée à l'exception de l'hydrogène, que ou bien l'on isole et si désiré, salifie, ou bien l'on fait réagir avec un amidure alcalin, puis avec un halogénure d'alcoyle de formule (IV):

Hal-R' (IV)

dans laquelle Hal représente un atome de chlore, de brome ou d'iode et R' représente un radical alcoyle renfermant de 1 à 4 atomes de carbone, pour obtenir un produit de formule ($I_D$):

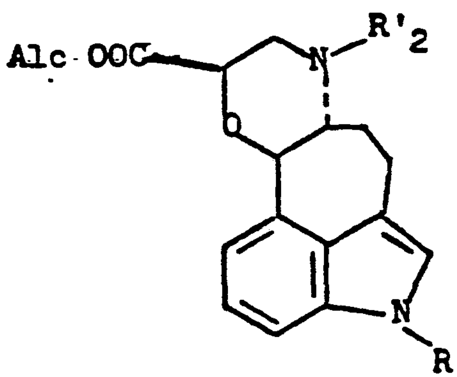

$(I_D)$

dans laquelle Alc, R' et $R'_2$ ont la signification déjà indiquée, que soit l'on isole et, si désiré, salifie, soit l'on soumet à l'action d'un agent d'halogénation pour obtenir un produit de formule $(I_E)$:

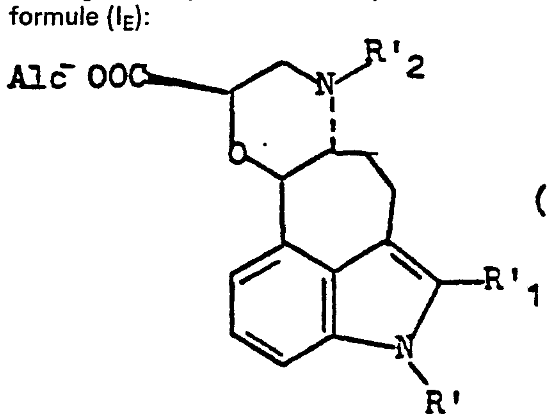

$(I_E)$

dans laquelle Alc, R' et $R'_2$ ont la signification déjà indiquée, et $R'_1$ représente un atome de chlore ou de brome que l'on isole et salifie si désiré, ou bien l'on soumet ledit produit de formule $(I_C)$ à l'action d'un agent d'halogénation pour obtenir un produit de formule $(I_F)$:

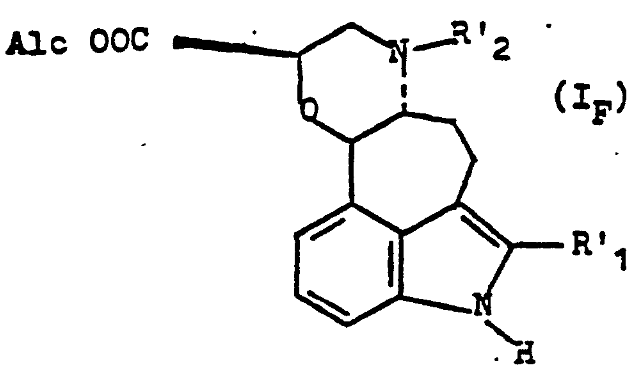

$(I_F)$

dans laquelle Alc, $R'_1$ et $R'_2$ ont la signification déjà indiquee, que l'on isole et si désiré, salifie, soit l'on saponifie ledit produit de formule $(I_A)$ pour obtenir un produit de formule$(I_G)$:

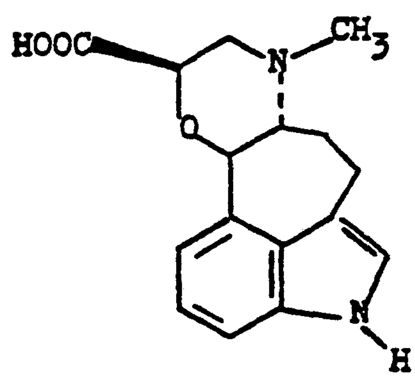

$(I_G)$

que soit l'on isole et, si désiré, salifie, soit l'on fait réagir avec une amine de formule (V):

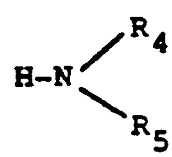

(V)

dans laquelle $R_4$ et $R_5$ ont la signification déjà indiquée, ou avec un peptide, pour obtenir un produit de formule $(I_H)$:

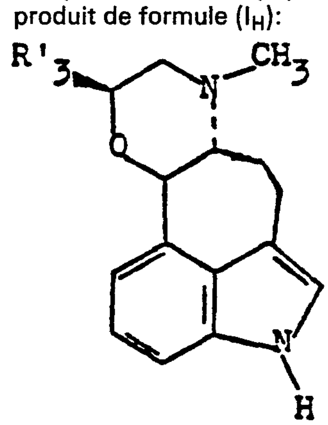

$(I_H)$

dans laquelle $R'_3$ représente un radical carboxy amidifié par l'amine de formule (V) définie ci-dessus, que l'on isole et si désiré, salifie, soit réduit ledit produit de formule $(I_A)$ pour obtenir un produit de formule $(I_I)$:

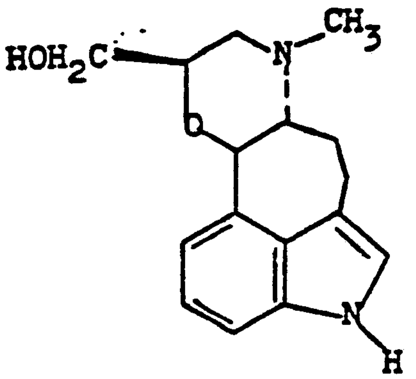

($I_I$)

que soit l'on isole et, si désiré, salifie, soit l'on fait réagir avec le chlorure de méthane ou de paratoluène sulfonyle pour obtenir un produit de formule (VI):

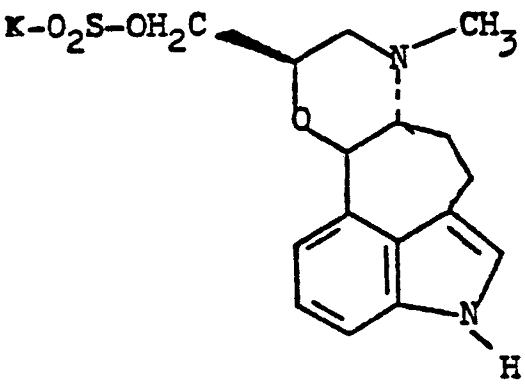

(VI)

dans laquelle K représente un radical méthyle ou p-tolyle, que l'on fait réagir avec un alcoylmercaptan ou avec un cyanure alcalin, pour obtenir un produit de formule ($I_J$):

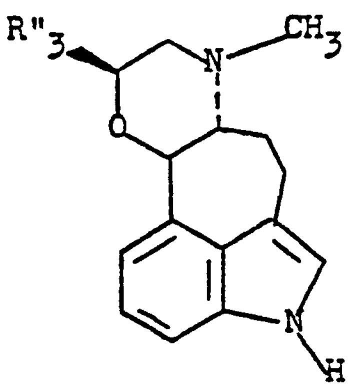

($I_J$)

dans laquelle R"$_3$ représente un radical alcoylthiométhyle ou cvanomethyle que l'on isole et si désiré, salifie, soit l'on oxyde le produit de formule ($I_A$), pour obtenir un produit de formule ($I_K$):

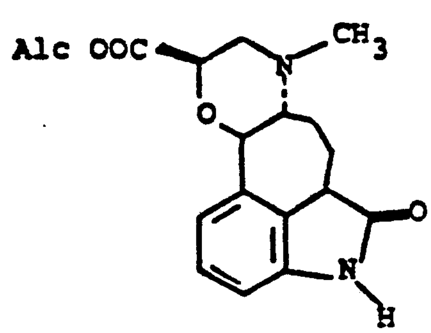

($I_K$)

dans laquelle Alc a la signification déjà indiquée, que ou bien l'on isole et, si désiré, salifie, ou bien l'on réduit pour obtenir un produit de formule ($I_L$):

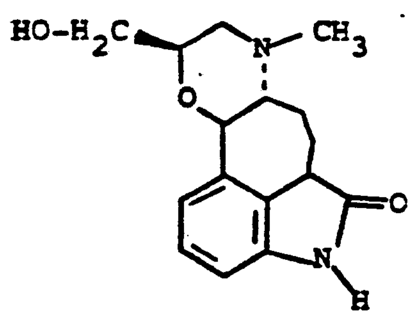

($I_L$)

que l'on isole et si désiré salifie, puis effectue ensuite si désiré, sur les produits de formules($I_F$, ($I_G$), ($I_H$), ($I_I$), ($I_J$), ($I_K$) et ($I_L$), une ou plusieurs des réactions déjà indiquées ci-dessus pour les produits de formule ($I_A$), pour obtenir les produits correspondants entrant dans la formule (I), puis isole, et si désiré, salifie les produits ainsi obtenus.

2.- procédé selon la revendication 1, pour la préparation des dérivés de la C-homo 9-oxaergoline répondant à la formule (I), dans laquelle a et b forment une double liaison, caractérisé en ce que l'on cyclise un produit de formule (II):

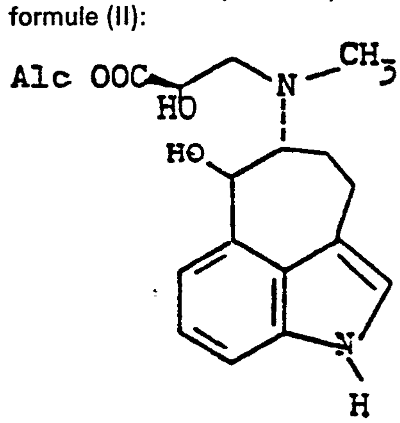

(II)

dans laquelle Alc représente un radical alcoyle renfermant de 1 à 4 atomes de carbone, pour obtenir un produit de formule (III);

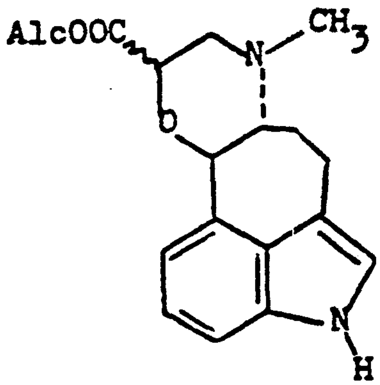

(III)

dans laquelle Alc a la signification déjà indiquée, et le trait ondulé signifie que le substituant est en position 8α ou 8β, dont, soit l'on isole l'isomère 8β, soit l'on épimé rise l'isomère 8α puis esterifie l'acide ainsi obtenu dans iequel le substituant en 8 est en position 8β, pour obtenir le produit de formule($I_A$):

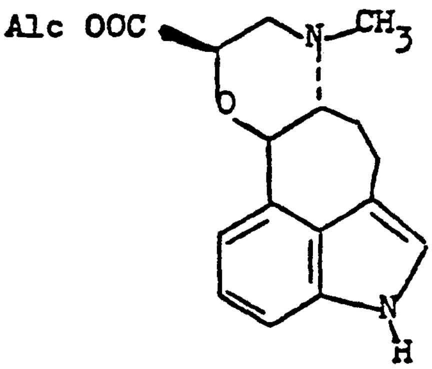

($I_A$)

dans laquelle Alc a la signification déjà indiquée, que

- soit l'on isole et si désiré, salifie,
- soit l'on déméthyle pour obtenir le produit de formule ($I_B$):

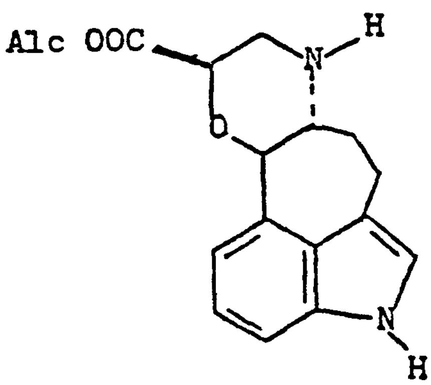

($I_B$)

dans laquelle Alc a la signification déjà indiquée, que soit l'on isole et si désiré, salifie, soit l'on fait réagir avec un agent d'alcoylation pour obtenir un produit de formule ($I_C$):

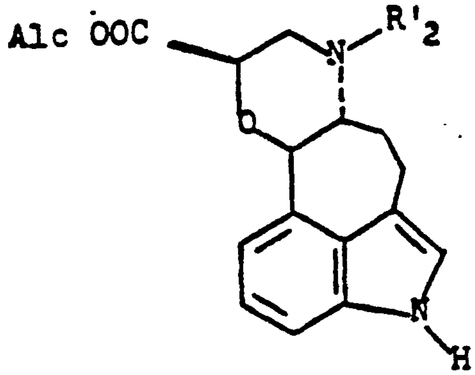

($I_C$)

dans laquelle $R'_2$ a la signification de $R_2$ déjà indiquée à l'exception de l'hydrogène, que ou bien l'on isole, et, si desire, salifie, ou bien l'on fait réagir avec un amidure alcalin, puis avec un halogénure d'alcoyle de formule (IV):

Hal-R' (IV)

dans laquelle Hal représente un atome de chlore, de brome ou d'iode et R' représente un radical alcoyle renfermant de 1 à 4 atomes de carbone, pour obtenir un produit de formule ($I_D$):

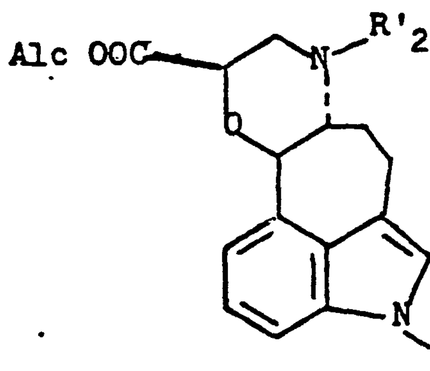

($I_D$)

dans laquelle Alc, R' et $R'_2$ ont la signification déjà indiquée, que soit l'on isole et, si désiré, salifie, soit l'on soumet à l'action d'un agent d'halogénation pour obtenir un produit de formule ($I_E$):

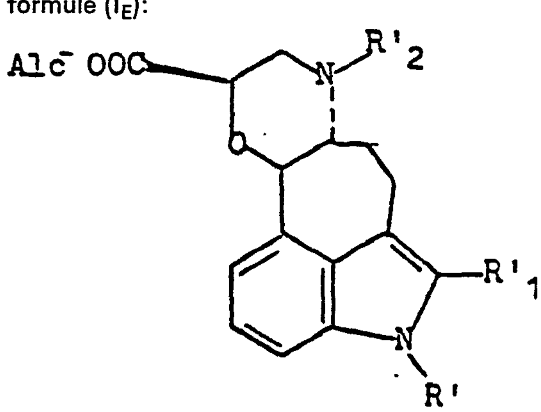

($I_E$)

dans laquelle Alc, R' et $R'_2$ ont la signification déjà indiquée, et $R'_1$ représente un atome de chlore ou de brome que l'on isole et salifie si désiré, ou bien l'on soumet ledit produit de formule ($I_C$) à l'action d'un agent d'halogénation pour obtenir un produit de formule ($I_F$):

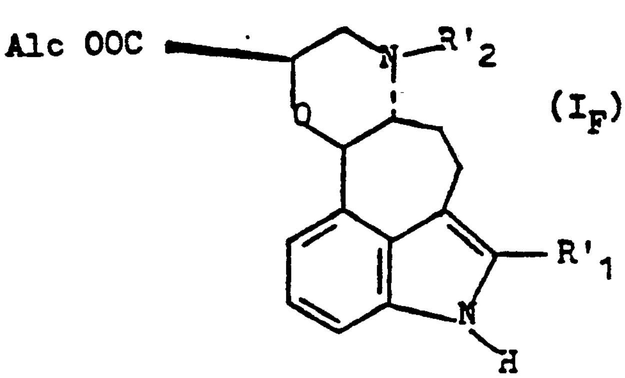

$(I_F)$

dans laquelle Alc, $R'_1$ et $R'_2$ ont la signification déjà indiquée, que l'on isole et si désiré, salifie, soit l'on saponifie ledit produit de formule $(I_A)$ pour obtenir un produit de formule$(I_G)$:

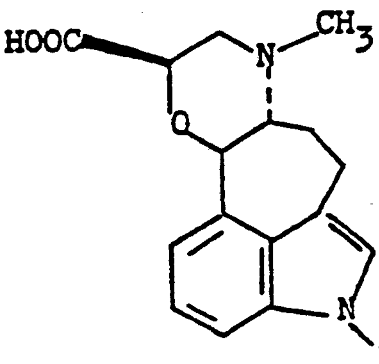

$(I_G)$

que soit l'on isole et, si désiré, salifie, soit l'on fait réagir avec une amine de formule (V):

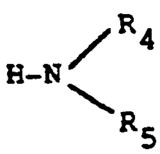

(V)

dans laquelle $R_4$ et $R_5$ ont la signification déjà indiquée, ou avec un peptide, pour obtenir un produit de formule $(I_H)$:

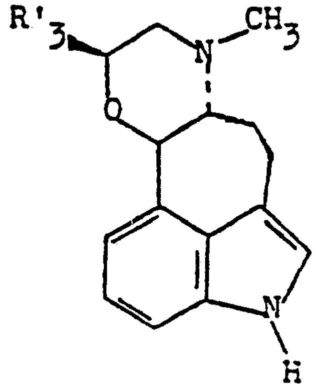

$(I_H)$

dans laquelle $R'_3$ représente un radical carboxy amidifié par l'amine de formule (V) définie ci-dessus, que l'on isole et si desiré, salifie, soit réduit ledit produit de formule $(I_A)$ pour obtenir un produit de formule $(I_I)$:

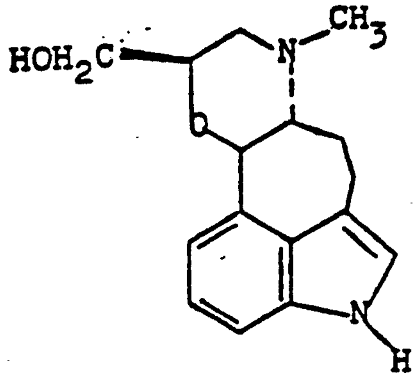

$(I_I)$

que soit l'on isole et, si désiré, salifie, soit l'on fait réagir avec le chlorure de méthane ou de paratoluène sulfonyle pour obtenir un produit de formule (VI):

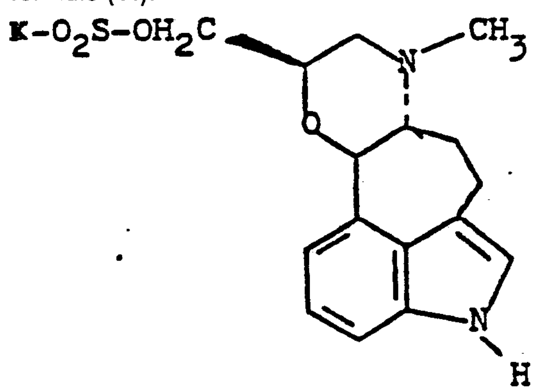

(VI)

dans laquelle K représente un radical méthyle ou p-tolyle, que l'on fait réagir avec un alcoylmercaptan ou avec un cyanure alcalin, pour obtenir un produit de formule $(I_J)$:

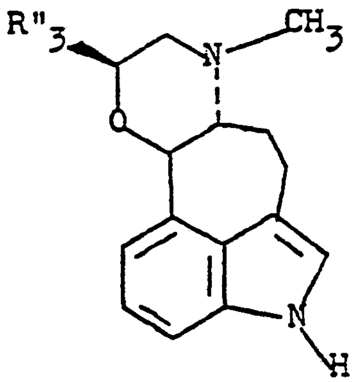

$(I_J)$

dans laquelle $R''_3$ représente un radical alcoylthiométhyle ou cyanométhyle que l'on isole et si désiré, salifie, puis effectue ensuite si désiré, sur les produits de formules $(I_F)$, $(I_G)$, $(I_H)$, $(I_I)$, $(I_J)$, une ou plusieurs des réactions déjà indiquees ci-dessus pour les produits de formule $(I_A)$, pour obtenir les produits correspondants entrant dans la formule (I), puis isole, et si désiré, salifie les produits ainsi obtenus.

3.- Procédé selon la revendication 2, caractérisé en ce que l'on utilise un agent d'alcoylation, permettant de fixer un radical $R_2$ renfermant de 1 à 4 atomes de carbone.

**Patentansprüche** für die Vertragsstaaten BE, CH, DE, FR, GB, IT, LI, LU, NL, SE

1. C-Homo-9-oxaergolin-Derivate und deren Additionssalze mit Mineral- oder organischen Säuren, dadurch gekennzeichnet, daß sie der allgemeinen Formel (I)

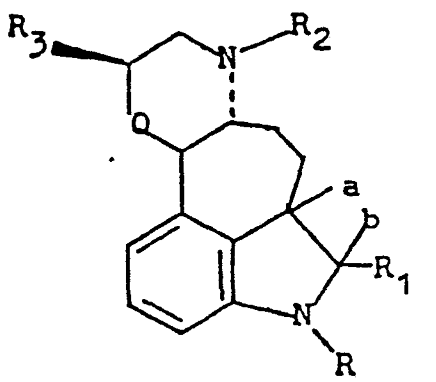

(I)

entsprechen, worin R ein Wasserstoffatom oder einen Alkylrest mit 1 bis 4 Kohlenstoffatomen bedeutet, $R_1$ ein Wasserstoff-, Chlor- oder Bromatom bedeutet oder mit b einen Oxorest bildet, a mit b eine Doppelbindung bildet oder a ein Wasserstoffatom bedeutet, wenn b mit $R_1$ einen Oxorest bildet, b mit a eine Doppelbindung bildet oder mit $R_1$ einen Oxorest bildet, $R_2$ ein Wasserstoffatom, einen Alkylrest mit 1 bis 4 Kohlenstoffatomen, einen Aralkylrest mit 7 bis 12 Kohlenstoffatomen oder einen Cycloalkalkylrest mit 4 bis 7 Kohlenstoffatomen bedeutet, $R_3$ einen Hydroxymethyl-, Alkylthiomethyl-, Cyanomethyl- oder Carboxyrest bedeutet, der gegebenenfalls mit einem aliphatischen Alkohol mit 1 bis 5 Kohlenstoffatomen verestert oder mit einem Amin der Formel

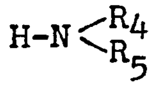

amidiert ist, worin $R_4$ ein Wasserstoffatom oder einen$^{5}$Alkylrest mit 1 bis 4 Kohlenstoffatomen bedeutet und R einen Alkylrest mit 1 bis 4 Kohlenstoffatomen bedeutet oder $R_4$ und $R_5$ gemeinsam mit dem Stickstoffatom einen gesättigten Meterocyclus, der gegebenenfalls ein weiteres Heteroatom enthält, bilden, wobei dieses letztgenamnte durch einen Alkylrest mit 1 bis 4 Kohlenstoffatomen substituiert sein kann, wenn der Heterocyclus ein weiteres Stickstoffatom enthält.

2. C-Homo-9-oxaergolin-Derivate und deren Additionssalze mit Mineral- oder organischen Säuren, dadurch gekennzeichnet, daß sie der Formel (I) gemäß Anspruch 1 entsprechen, worin a und b eine Doppelbindung bilden.

3. C-Homo-9-oxaergolin-Derivate und deren Additionssalze mit Mineral- oder organischen Säuren, dadurch gekennzeichnet, daß sie der Formel (1) gemäß Anspruch 2 entsprechen, worin R ein Wasserstoffatom bedeutet.

4. C-Homo-9-oxaergolin-Derivate gemäß Anspruch 3 und deren Additionssalze mit Mineral- oder organischen Säuren, dadurch gekennzeichnet, daß $R_1$ ein Wasserstoff-, Chlor- oder Bromatom bedeutet, $R_2$ einen Alkylrest mit 1 bis 4 Kohlenstoffatomen bedeutet, und $R_3$ einen Hydroxymethyl-, Methylthiomethyl- oder Cyanomethylrest oder einen Carboxylrest bedeutet, der gegebenenfalls mit einem linearen, aliphatischen Alkohol mit 1 bis 4 Kohlenstoffatomen verestert oder mit einem Amin der Formel

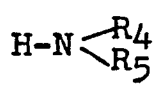

amidiert ist, worin $R_4$ und $R_5$ einen linearen Al kylrest mit 1 bis 4 Kohlenstoffatomen bedeuten.

5. Eines der Produkte gemäß Anspruch 1 mit den folgenden Bezeichnungen:
Ethyl-[5-RS-(5α,8β,10β)]-6-methyl-C-homo-9-oxa-ergolin-8-carboxylat
Methyl-[5-RS-(5α,8β,10β)]-6-methyl-C-homo-9-oxa-ergolin-8-carboxylat und
[5-RS-(5α,8β,10β)]-6-Methyl-C-homo-9-oxaergolin-8-methanol sowie deren Additionssalze mit Mineral- oder organischen Säuren.

6. Verfahren zur Herstellung der C-Homo-9-oxa-ergolin-Derivate der Formel (I) gemäß Anspruch 1 und von deren Salzen, dadurch gekennzeichnet, daß man ein Produkt der Formel (II)

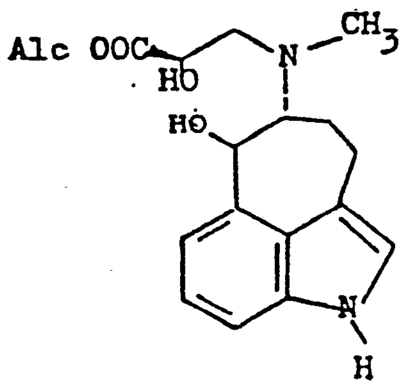

(II)

worin Alc einen Alkylrest mit 1 bis 4 Kohlenstoffatomen bedeutet, cyclisiert, um ein Produkt der Formel (III)

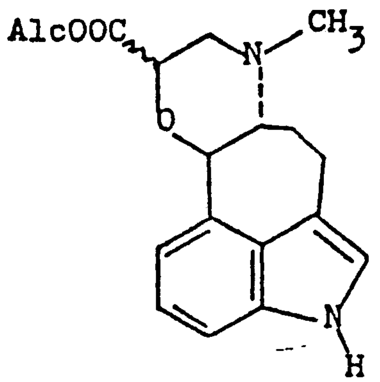

(III)

zu erhalten, worin Alc die angegebene Bedeutung besitzt und die gewellte Linie anzeigt, daß sich der Substituent in 8α- oder 8ß-Position befinden kann, wovon man entweder das 8ß-Isomere isoliert oder das 8α-Isomere epimerisiert, danach die so erhaltene Säure, in der sich der Substituent in 8-Stellung in 8β-Position befindet, verestert, um das Produkt der Formel ($I_A$)

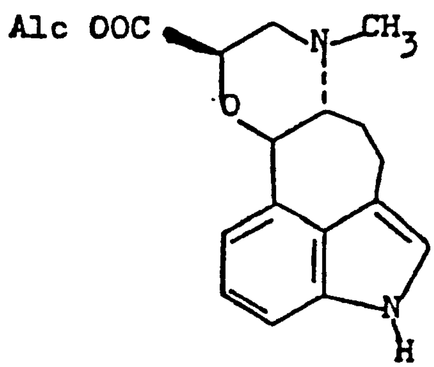

($I_A$)

zu erhalten, worin Alc die angegebene Bedeutung besitzt, das man

entweder isoliert und gewünschtemfalls in ein Salz überführt

oder entmethyliert, um das Produkt der Formel ($I_B$)

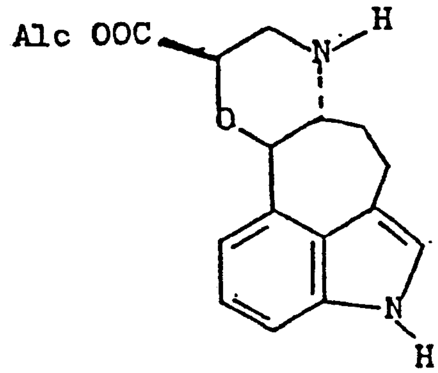

($I_B$)

zu erhalten, worin Alc die angegebene Bedeutung besitzt, das man entweder isoliert und gewünschtenfalls in ein Salz überführt oder mit einem Alkylierungsmittel umsetzt, um ein Produkt der Formel ($I_C$)

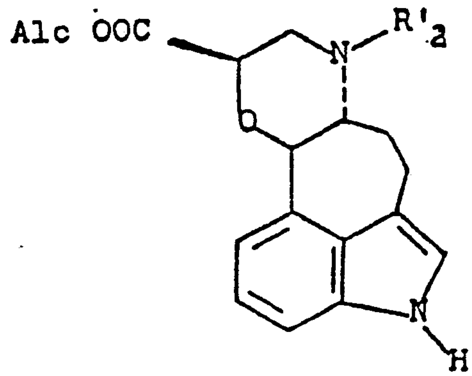

($I_C$)

zu erhalten, worin $R'_2$ die für $R_2$ angegebene Bedeutung mit Ausnahme von Wasserstoff besitzt, das man entweder isoliert und gewünschtenfalls in ein Salz überführt oder mit einem Alkaliamid und danach mit einem Alkylhalogenid der Formel (IV)

Hal-R' (IV)

umsetzt, worin Hal ein Chlor-, Brom- oder Jodatom bedeutet und R' einen Alkylrest mit 1 bis 4 Kohlenstoffatomen bedeutet, um ein Produkt der Formel ($I_D$)

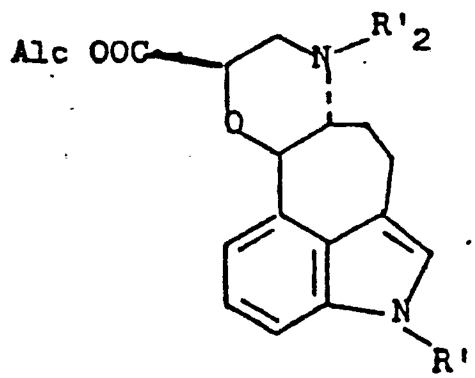

($I_D$)

zu erhalten, worin Alc, R' und $R'_2$ die angegebene Bedeutung besitzen, das man entweder isoliert und gewünsch tenfalls in ein Salz überführt oder der Einwirkung eines Hälogenierungsmittels unterzieht, um ein Produkt der Formel ($I_E$)

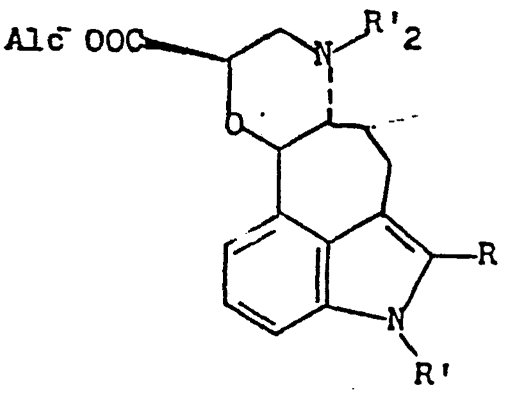

($I_E$)

zu erhalten, worin Alc, R' und $R'_2$ die angegebene Bedeutung besitzen und $R'_1$ ein Chlor- oder Bromatom bedeutet, welches man isoliert und gewünschtenfalls in ein Salz überführt, oder das Produkt der Formel ($I_C$) der Einwirkung eines Halogenierungsmittels unterzieht, um ein Produkt der Formel ($I_F$)

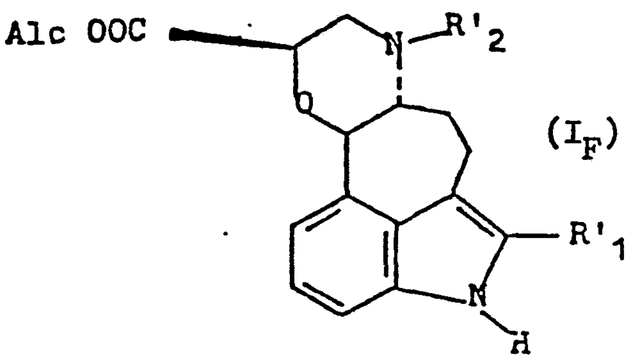

($I_F$)

zu erhalten, worin Alc, $R'_1$ und $R'_2$ die angegebene Bedeutung besitzen, das man isoliert und gewünschtenfalls in ein Salz überführt,

<u>oder</u> das Produkt der Formel ($I_A$) verseift, um ein Produkt der Formel ($I_G$)

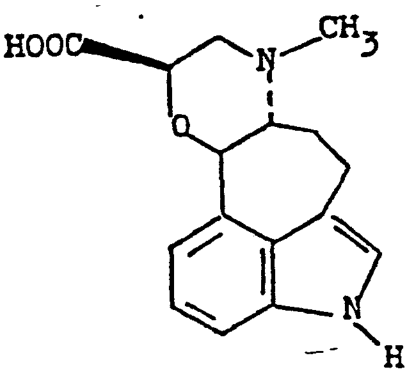

($I_G$)

entweder

zu erhalten, das man entweder isoliert und gewünschtenfalls in ein Salz überführt oder mit einem Amin der Formel (V)

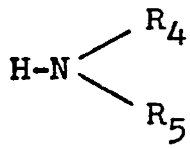

(V)

worin $R_4$ und $R_5$ die angegebene Bedeutung besitzen, oder mit einem Peptid umsetzt, um ein Produkt der Formel ($I_H$)

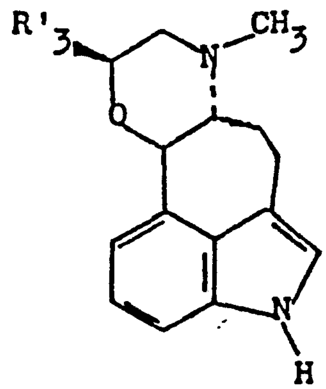

($I_H$)

zu erhalten, worin $R'_3$ einen durch das Amin der vorstehend definierten Formel (V) amidierten Carboxyrest bedeutet, welches man isoliert und gewünschtenfalls in ein Salz überführt,

<u>oder</u> das Produkt der Formel ($I_A$) reduziert, um ein Produkt der Formel ($I_I$)

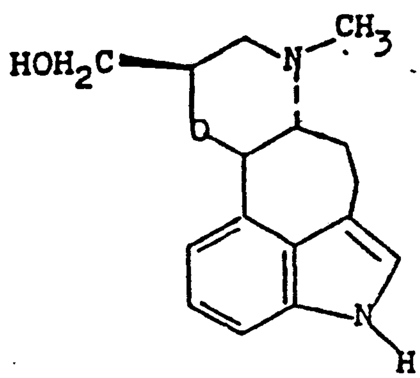

($I_I$)

zu erhalten, das man entweder isoliert.und gewünschtenfalls in ein Salz überführt oder mit Methansulfonylchlorid oder p-Toluolsulfonylchlorid umsetzt, um ein Produkt der Formel (VI)

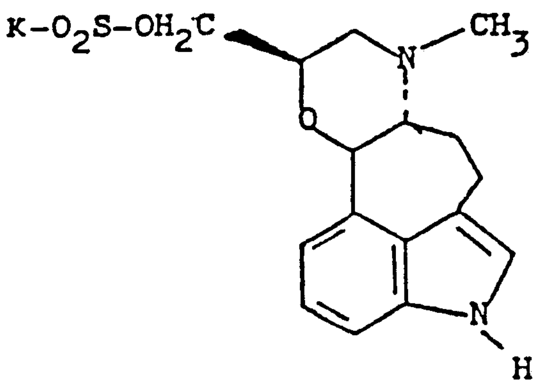

(VI)

zu erhalten, worin K einen Methyl- oder p-Tolylrest bedeutet, welches man mit einem Alkylmercaptan oder mit einem Alkalicyanid umsetzt, um ein Produkt der Formel ($I_J$)

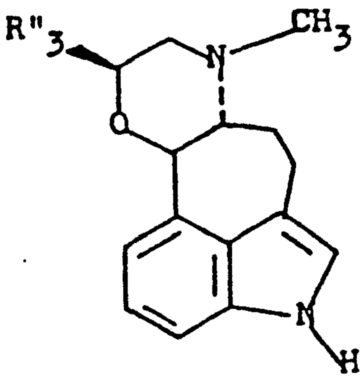

($I_J$)

zu erhalten, worin R"$_3$ einen Alkylthiomethyl- oder Cyanomethylrest bedeutet, welches man isoliert und gewünschtenfalls in ein Salz überführt, <u>oder</u> das Produkt der Formel ($I_A$) oxidiert, um ein Produkt der Formel ($I_K$)

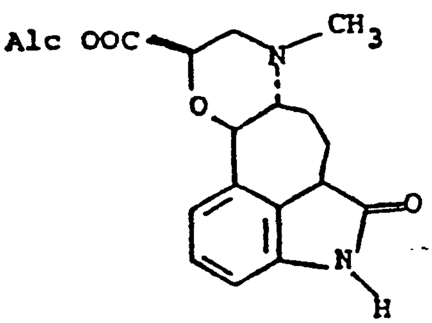

($I_K$)

zu erhalten, worin Alc die angegebene Bedeutung besitzt, das man entweder isoliert und gewünschtenfalls in ein Salz überführt oder reduziert, um ein Produkt der Formel ($I_L$)

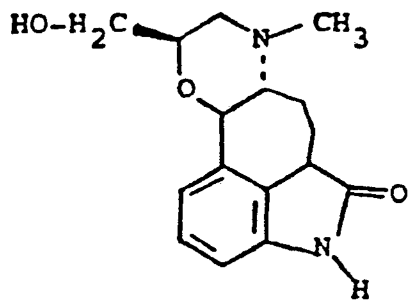

($I_L$)

zu erhalten, das man isoliert und gewünschtenfalls in ein Salz überführt, wonach man gewünschtenfalls an-schließend an den Produkten der Formeln ($I_F$), ($I_G$), ($I_H$), ($I_I$), ($I_J$), ($I_K$) und ($I_L$) eine oder mehrere der vorstehend für die Produkte der Formel ($I_A$) angegebenen Reaktionen durchführt, um die entsprechenden, unter die Formel (I) fallenden Produkte zu erhalten, und an-schließend die so erhaltenen Produkte isoliert und gewünschtenfalls in ein Salz überführt.

7. Arzneimittel, dadurch gekennzeichnet, daß sie aus den neuen C-Homo-9-oxaergolin-Derivaten der Formel (I) gemäß Anspruch 1 sowie aus deren Additionssalzen mit pharmazeutisch verträglichen Säuren bestehen.

8. Arzneimittel, dadurch gekennzeichnet, daß sie aus den neuen C-Homo-9-oxaergolin-Derivaten gemäß Anspruch 2 sowie aus deren Additionssalzen mit pharmazeutisch verträglichen Säuren bestehen.

9. Arzneimittel, dadurch gekennzeichnet, daß sie aus den neuen C-Homo-9-oxaergolin-Derivaten gemäß Anspruch 3 oder 4 sowie aus deren Additionssalzen mit pharmazeutisch verträglichen Säuren bestehen.

10. Arzneimittel, dadurch gekennzeichnet, daß sie aus den C-Homo-9-oxaergolin-Derivaten gemäß Anspruch 5 sowie aus deren Additionssalzen mit pharmazeutisch verträglichen Säuren bestehen.

11. Pharmazeutische Zusammensetzungen, dadurch gekennzeichnet, daß sie als Wirkstoff zumindest eines der Arzneimittel gemäß einem der Ansprüche 7 bis 10 enthalten.

12. Als neue, für die Herstellung der Derivate der Formel (I) gemäß Anspruch 1 erforderliche, industrielle Produkte die Derivate der Formel (II)

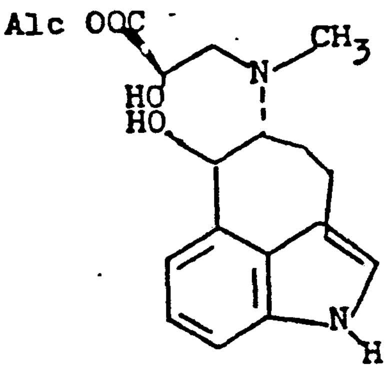

(II)

worin Alc die in Anspruch 6 angegebene Bedeutung besitzt.

**Patentansprüche** für den Vertragsstaat: AT

1. Verfahren zur Herstellung neuer C-Homo-9-oxaergolin-Derivate und von deren Additionssalzen mit Mineral- oder organischen Säuren der allgemeinen Formel (I)

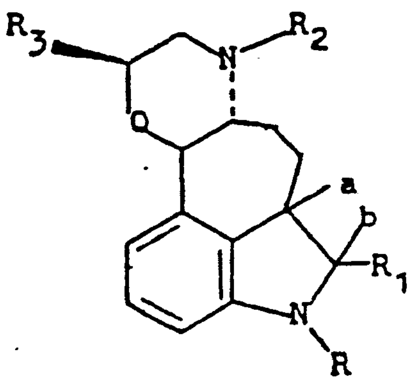

(I)

worin R ein Wasserstoffatom oder einen Alkylrest mit 1 bis 4 Kohlenstoffatomen bedeutet, $R_1$ ein Wasserstoff-, Chlor- oder Bromatom bedeutet oder mit b einen Oxorest bildet, a mit b eine Doppelbindung bildet oder a ein Wasserstoffatom bedeutet, wenn b mit $R_1$ einen Oxorest bildet, b mit a eine Doppelbindung bildet oder mit $R_1$ einen Oxorest bildet, $R_2$ ein Wasserstoffatom, einen Alkylrest mit 1 bis 4 Kohlenstoffatomen, einen Aralkylrest mit 7 bis 12 Kohlenstoffatomen oder einen Cycloalkalkylrest mit 4 bis 7 Kohlenstoffatomen bedeutet, $R_3$ einen Hydroxymethyl-, Alkylthiomethyl—, Cyanomethyl- oder Carboxyrest bedeutet, der gegebenenfalls mit einem aliphatischen Alkohol mit 1 bis 5 Kohlenstoffatomen verestert oder mit einem Amin der Formel

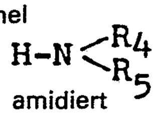

amidiert

ist, worin $R_4$ ein Wasserstoffatom oder einen Alkylrest mit 1 bis 4 Kohlenstoffatomen bedeutet und R einen Alkylrest mit 1 bis 4 Kohlenstoffatomen bedeutet oder $R_4$ und $R_5$ gemeinsam mit dem Stickstoffatom einen gesättigten Heterocyclus, der gegebenenfalls ein weiteres Heteroatom enthält, bilden, wobei dieses letztere durch einen Alkylrest mit 1 bis 4 Kohlenstoffatomen substituiert sein kann, wenn der Heterocyclus ein weiteres Stickstoffatom enthält,

dadurch gekennzeichnet, daß man ein Produkt der Formel (II)

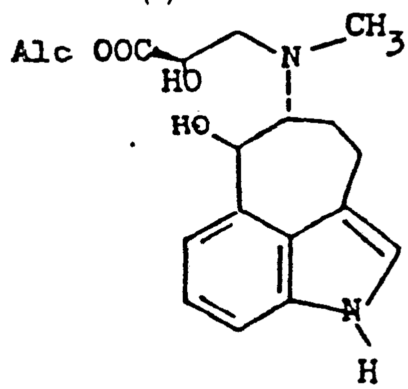

(II)

worin Alc einen Alkylrest mit 1 bis 4 Kohlenstoffatomen bedeutet, cyclisiert, um ein Produkt der Formel (III)

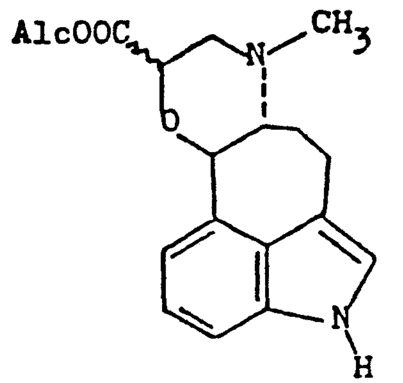

(III)

zu erhalten, worin Alc die angegebene Bedeutung besitzt und die gewellte Linie anzeigt, daß sich der Substituent in 8α- oder 8ß-Position befinden kann, wovon man entweder das 8ß-Isomere isoliert oder das 8α-Isomere epimerisiert, danach die so erhaltene Säure, in der sich der Substituent in 8-Stellung in 8ß-Position befindet, verestert, um das Produkt der Formel ($I_A$)

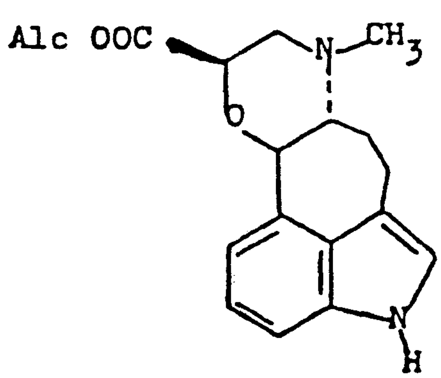

($I_A$)

zu erhalten, worin Alc die angegebene Bedeutung besitzt, das man

entweder isoliert und gewünschtenfalls in ein Salz überführt

<u>oder</u> entmethyliert, um das Produkt der Formel ($I_B$)

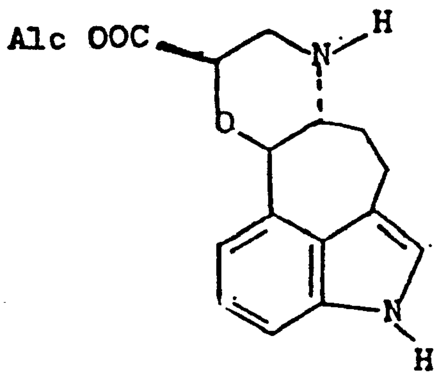

($I_B$)

zu erhalten, worin Alc die angegebene Bedeutung besitzt, das man entweder isoliert und gewünschtenfalls in ein Salz überführt oder mit einem Alkylierungsmittel umsetzt, um ein Produkt der Formel ($I_C$)

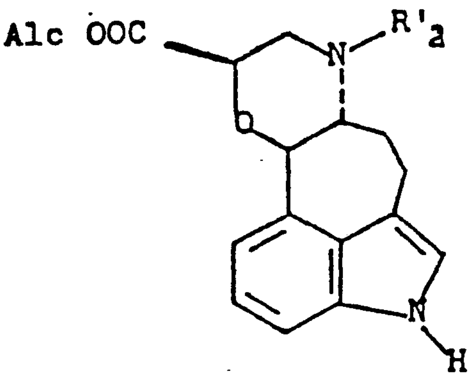

($I_C$)

zu erhalten, worin $R'_2$ die für $R_2$ angegebene Bedeutung mit Ausnahme von Wasserstoff besitzt, das man <u>entweder</u> isoliert und gewünschtenfalls in ein Salz überführt <u>oder</u> mit einem Alkaliamid und danach mit einem Alkylhalogenid der Formel (IV)

Hal-R' (IV)

umsetzt, worin Hal ein Chlor-, Brom- oder Jodatom bedeutet und R' einen Alkylrest mit 1 bis 4 Kohlenstoffatomen bedeutet, um ein Produkt der Formel ($I_D$)

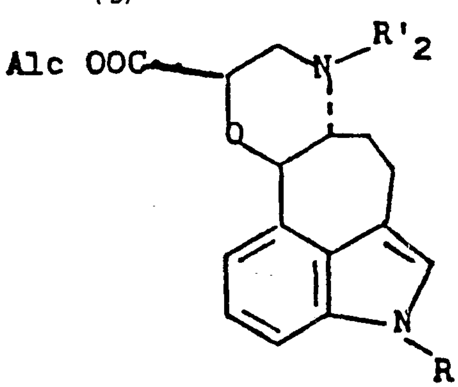

($I_D$)

zu erhalten, worin Alc, R' und $R'_2$ die angegebene Bedeutung besitzen, das man entweder isoliert und gewünschtenfalls in ein Salz überführt oder der Einwirkung eines Hälogenierungsmittels unterzieht, um ein Produkt der Formel ($I_E$)

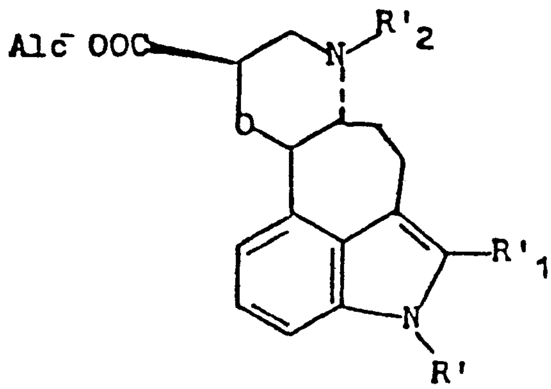

($I_E$)

zu erhalten, worin Alc, R' und $R'_2$ die angegebene Bedeutung besitzen und $R'_1$ ein Chlor- oder Bromatom bedeutet, wejches man isoliert und gewünschtenfalls in ein Salz überführt, <u>oder</u> das Produkt der Formel ($I_C$) der Einwirkung eines Halogenierungsmittels unterzieht, um ein Produkt der Formel ($I_F$)

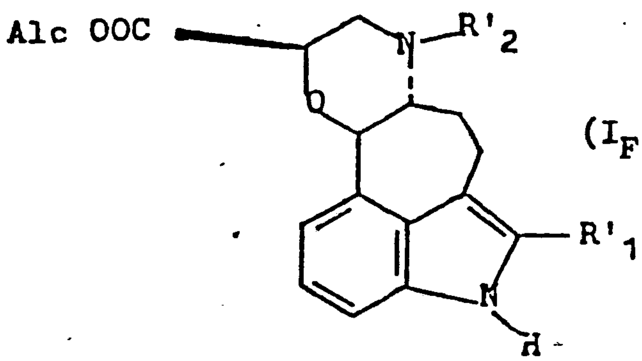

($I_F$)

zu erhalten, worin Alc, $R'_1$ und $R'_2$ die angegebene Bedeutung besitzen, das man isoliert und gewünschtenfalls in ein Salz überführt,

<u>oder</u> das Produkt der Formel ($I_A$) verseift, um ein Produkt der Formel ($I_G$)

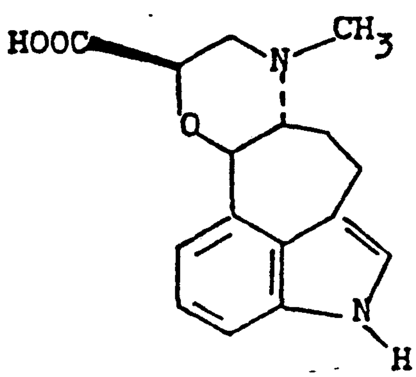

($I_G$)

zu erhalten, das man entweder isoliert und gewünschtenfalls in ein Salz überführt oder mit einem Amin der Formel (V)

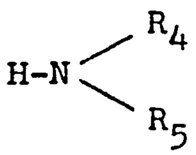

(V)

worin $R_4$ und $R_5$ die angegebene Bedeutung besitzen, oder mit einem Peptid umsetzt, um ein Produkt der Formel ($I_H$)

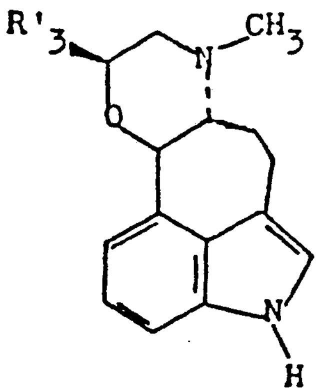

($I_H$)

zu erhalten, worin $R'_3$ einen durch das Amin der vorstehend definierten Formel (V) amidierten Carboxyrest bedeutet, welches man isoliert und gewünschtenfalls in ein Salz überführt,

<u>oder</u> das Produkt der Formel ($I_A$) reduziert, um ein Produkt der Formel ($I_I$)

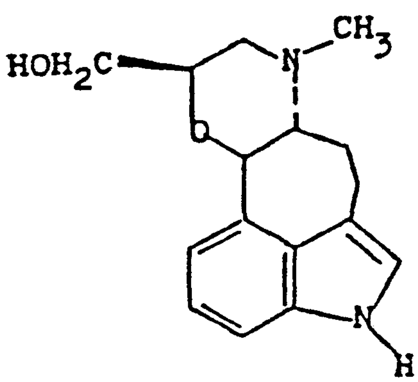

($I_I$)

zu erhalten, das man entweder isoliert und gewünschtenfalls in ein Salz überführt oder mit Methansulfonylchlorid oder p-Toluolsulfonylchlorid umsetzt, um ein Produkt der Formel (VI)

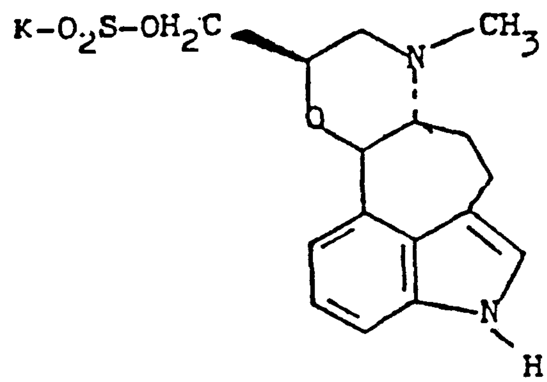

(VI)

zu erhalten, worin K einen Methyl- oder p-Tolylrest bedeutet, welches man mit einem Alkylmercaptan oder mit einem Alkalicyanid umsetzt, um ein Produkt der Formel ($I_J$)

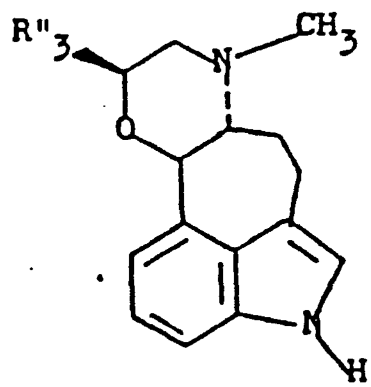

($I_J$)

zu erhalten, worin $R''_3$ einen Alkylthiomethyl- oder Cyanomethylrest bedeutet, welches man isoliert und gewünschtenfalls in ein Salz überführt,

<u>oder</u> das Produkt der Formel ($I_A$) oxidiert, um ein Produkt der Formel ($I_K$)

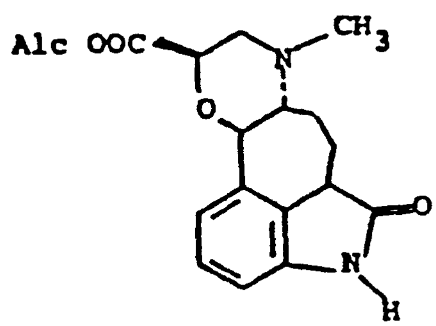

($I_K$)

zu erhalten, worin Alc die angegebene Bedeutung besitzt, das man entweder isoliert und gewünschtenfalls in ein Salz überführt oder reduziert, um ein Produkt der Formel ($I_L$)

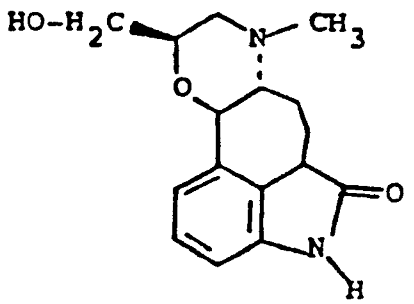

$(I_L)$

zu erhalten, das man isoliert und gewünschtenfalls in ein Salz überführt, wonach man gewünschtenfalls an-schließend an den Produkten der Formeln $(I_F)'$ $(I_G)'$ $(I_H)$, $(I_I)$, $(I_J)$, $(I_K)$ und $(I_L)$ eine oder mehrere der vorstehend für die Produkte der Formel $(I_A)$ angegebenen Reaktionen durchführt, um die entsprechenden, unter die Formel (I) fallenden Produkte zu erhalten, und an-schließend die so erhaltenen Produkte isoliert und gewünschtenfalls in ein Salz überführt.

2. Verfahren gemäß Anspruch 1 zur Herstellung der C-Homo-9-oxaergolin-Derivate der Formel (I), worin a und b eine Doppelbindung bilden, dadurch gekennzeichnet, daß man ein Produkt der Formel (II)

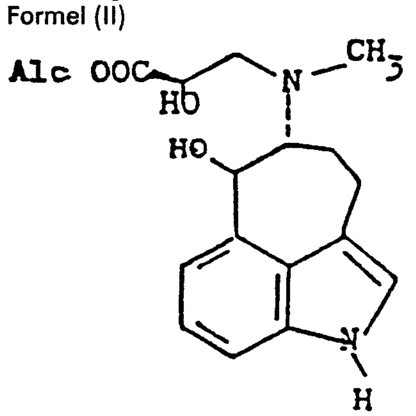

(II)

worin Alc einen Alkylrest mit 1 bis 4 Kohlenstoffatomen bedeutet, cyclisiert, um ein Produkt der Formel (III)

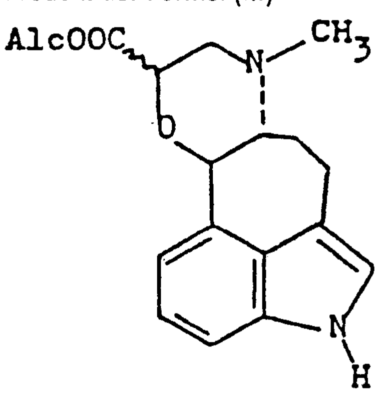

(III)

zu erhalten, worin Alc die angegebene Bedeutung besitzt und die gewellte Linie anzeigt, daß sich der Substituent in 8α- oder 8ß-Position befindet, wovon man entweder das 8ß-Isomere isoliert oder das 8α-Isomere epimerisiert, dann die so erhaltene Säure, in der sich der ß-Substituent in 8ß-Position befindet, verestert, um das Produkt der Formel $(I_A)$

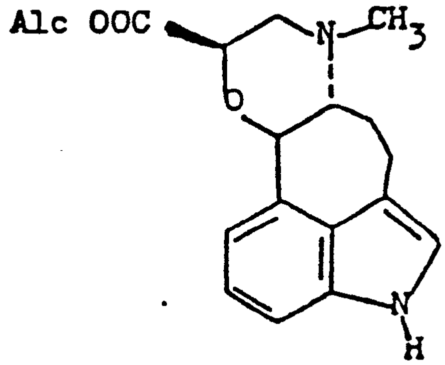

$(I_A)$

zu erhalten, worin Alc die angegebene Bedeutung besitzt, das man

entweder isoliert und gewünschtenfalls in ein Salz überführt

oder entmethyliert, um das Produkt der Formel $(I_B)$

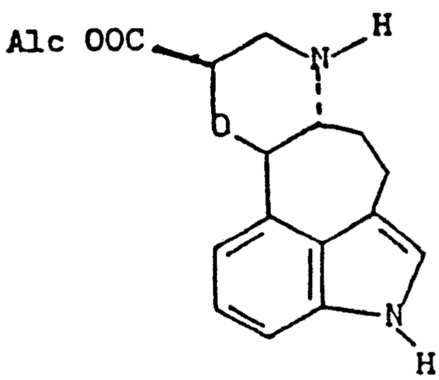

$(I_B)$

zu erhalten, worin Alc die angegebene Bedeutung besitzt, das man entweder isoliert und gewünschtenfalls in ein Salz überführt oder mit einem Alkylierungsmittel umsetzt um ein Produkt der Formel $(I_C)$

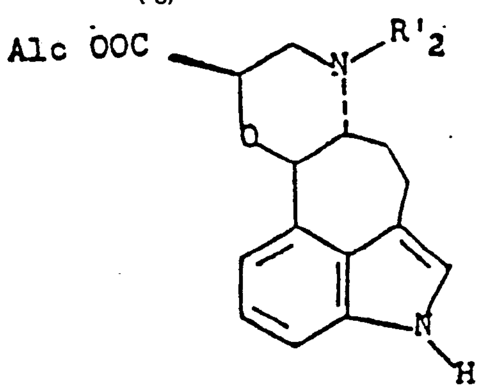

$(I_C)$

zu erhalten, worin $R'_2$ die für $R_2$ angegebene Bedeutung mit Ausnahme von Wasserstoff besitzt, das man entweder isoliert und gewünschtenfalls in ein Salz überführt oder mit einem Alkaliamid und danach mit einem Alkylhalogenid der Formel (IV)

Hal-R' (IV)

umsetzt, worin Hal ein Chlor-, Brom- oder

Jodatom bedeutet und R' einen Alkylrest mit 1 bis 4 Kohlenstoffatomen bedeutet, um ein Produkt der Formel ($I_D$)

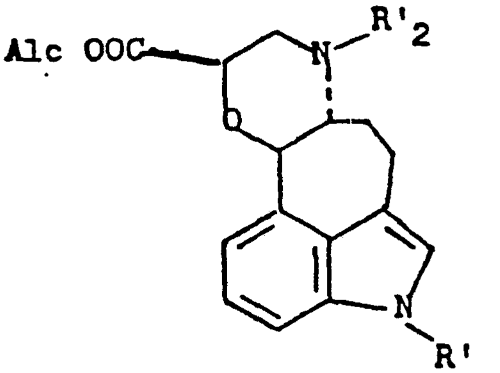

($I_D$)

zu erhalten, worin Alc, R' und $R'_2$ die angegebene Bedeutung besitzen, das man entweder isoliert und gewünschtenfalls in ein Salz überführt oder der Einwirkung eines Hälogenierungsmittels unterzieht, um ein Produkt der Formel ($I_E$)

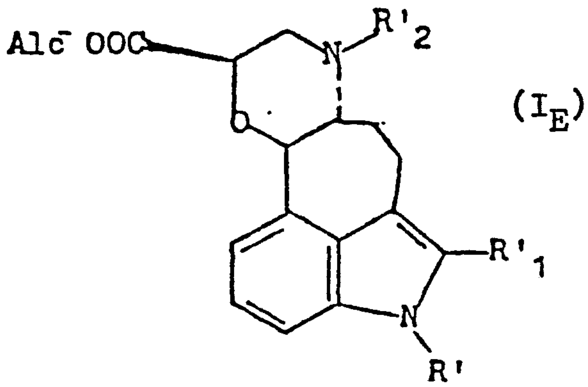

($I_E$)

zu erhalten, worin Alc, R' und $R'_2$ die angegebene Bedeutung besitzen und $R'_1$ ein Chlor- oder Bromatom bedeutet, welches man isoliert und gewünschtenfalls in ein Salz überführt, <u>oder</u> das Produkt der Formel ($I_C$) der Einwirkung eines Halogenierungsmittels unterzieht, um ein Produkt der Formel ($I_F$)

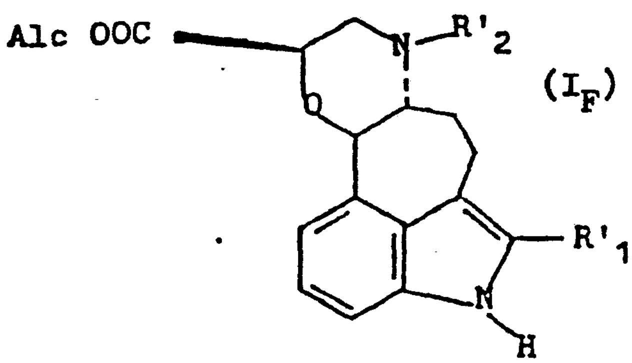

($I_F$)

zu erhalten, worin Alc, $R'_1$ und $R'_2$ die angegebene Bedeutung besitzen, das man isoliert und gewünschtenfalls in ein Salz überführt,

<u>oder</u> das Produkt der Formel ($I_A$) verseift, um ein Produkt der Formel ($I_G$)

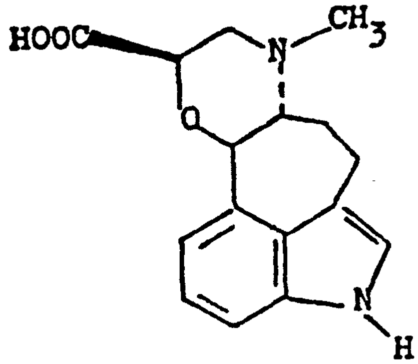

($I_G$)

zu erhalten, das man entweder isoliert und gewünschtenfalls in ein Salz überführt oder mit einem Amin der Formel (V)

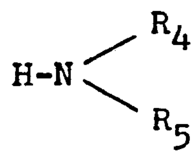

(V)

worin $R_4$ und $R_5$ die angegebene Bedeutung besitzen, oder mit einem Peptid umsetzt, um ein Produkt der Formel ($I_H$)

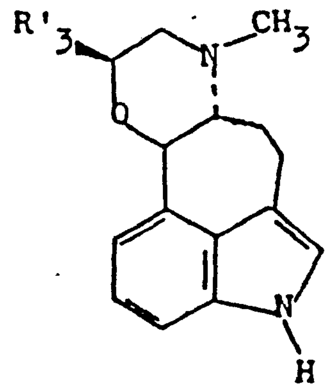

($I_H$)

zu erhalten, worin $R'_3$ eÜlnen durch das Amin der vorstehend definierten Formel (V) amidierten Carboxyrest bedeutet, welches man isoliert und gewünschtenfalls in ein Salz überführt,

<u>oder</u> das Produkt der Formel ($I_A$) reduziert, um ein Produkt der Formel ($I_I$)

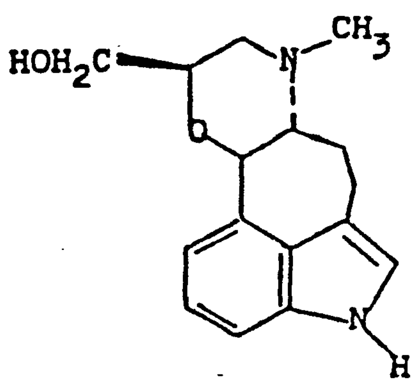

($I_I$)

zu erhalten, das man entweder isoliert und gewünschtenfalls in Salz überführt oder mit Methansulfonylchlorid oder p-Toluolsulfonylchlorid umsetzt, um ein Produkt der Formel (VI)

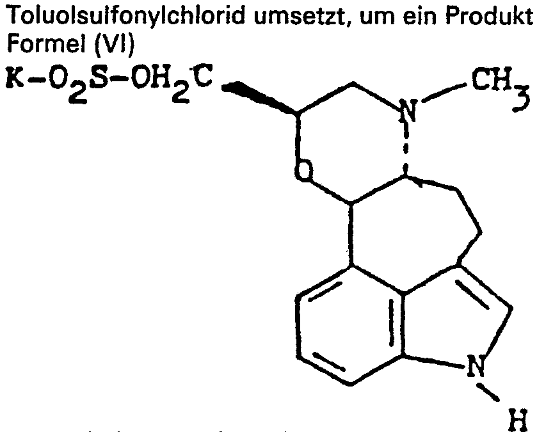

(VI)

zu erhalten, worin K einen Methyl- oder p-Tolylrest bedeutet, welches man mit einem Alkylmercaptan oder mit einem Alkalicyanid umsetzt, um ein Produkt der Formel ($I_J$)

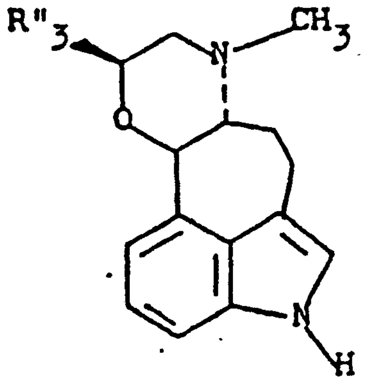

($I_J$)

zu erhalten, worin $R''_3$ einen Alkylthiomethyl- oder Cyanomethylrest bedeutet, welches man isoliert und gewünscntenfalls in ein Salz überführt, wonach man an-schließend gewünschtenfalls an den Produkten der Formeln ($I_F$), ($I_G$), ($I_H$), ($I_I$), ($I_J$) eine oder mehrere der vorstehend für die Produkte der Formel ($I_A$) angegebenen Reaktionen durchführt, um die entsprechenden, unter die Formel (I) fallenden Produkte zu erhalten, und dann die so erhaltenen Produkte isoliert und gewünschtenfalls in ein Salz überführt.

3. Verfahren gemäß Anspruch 2, dadurch gekennzeichnet, daß man ein Alkylierungsmittel verwendet, das es ermöglicht, einen Rest $R_2$ zu fixieren, der 1 bis 4 Kohlenstoffatome enthält.

**Claims** for the Contracting states BE CH DE FR GB IT LI LU NL SE

1) Derivatives of C-homo-9-oxaergoline, as well as their salts of addition with mineral or organic acids, characterized in that they answer to the general formula (I):

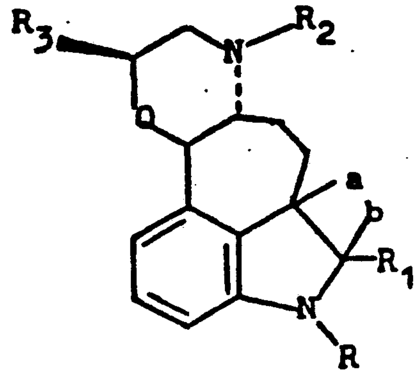

(I)

in which R represents a hydrogen atom or an alkyl radical containing from 1 to 4 carbon atoms, $R_1$ represents a hydrogen, chlorine or bromine atom or forms with b an oxo radical, a forms with b a double bond or a represents a hydrogen atom when b forms with $R_1$ an oxo radical, b forms with a a double bond or forms with $R_1$ an oxo radical, $R_2$ represents a hydrogen atom, an alkyl radical contäining from 1 to 4 carbon atoms, an aralkyl radical containing from 7 to 12 carbon atoms, or a cycloalkylalkyl radical containing from 4 to 7 carbon atoms, $R_3$ represents a hydroxymethyl, alkylthiomethyl, cyanomethyl or carboxy radical possibly esterified by an aliphatic alcohol containing from 1 to 5 carbon atoms, or amidified by an amine with the formula

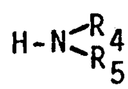

in which $R_4$ represents a hydrogen atom or an alkyl radical containing from 1 to 4 carbon atoms, and $R_5$ represents an alkyl radical containing from 1 to 4 carbon atoms or $R_4$ and $R_5$ form together with the nitrogen atom a saturated heterocycle possibly containing another heteroatom, which latter can be substituted by an alkyl radical containing from 1 to 4 carbon atoms when the heterocycle contains another nitrogen atom.

2) Derivatives of C-homo-9-oxaergoline as well as their salts of addition with mineral or organic acids, characterized in that they answer to the formula (I) of claim 1, in which a and b form a double bond.

3) Derivatives of C-homo-oxaergoline, as well as their salts of addition with mineral or organic acids, characterized in that they answer to the formula (I) of claim 2, in which R represents a hydrogen atom.

4) Derivatives of C-homo-9-oxaergoline according to claim 3, as well as their salts of addition with mineral or organic acids, characterized in that $R_1$ represents a hydrogen, chlorine or bromine atom, $R_2$ represents an alkyl radical containing from 1 to 4 carbon atoms and $R_3$ represents a hydroxymethyl, methylthiomethyl or cyanomethyl radical, a carboxy radical possibly esterified by a linear aliphatic alcohol containing from 1 to 4 carbon atoms or amidified by an amine with the formula

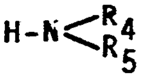

in which $R_4$ and $R_5$ represent a linear alkyl radical containing from 1 to 4 carbon atoms.

5) Any one of the products according to claim 1, the names of which follow:

- ethyl [5 RS (5α, 8β, 10β)]-6-methyl-C-homo-9-oxaergoline-8-carboxylate;
- methyl [5 RS (5α, 8β, 10β)]-6-methyl-C-homo-9-oxaergoline-8-carboxylate and
- [5 RS (5α, 8β, 10β)]-6-methyl-C-homo-9-oxaergoline-8-methanol,

as well as their salts of addition with mineral or organic acids.

6) Preparation process of derivatives of C-homo-9-oxaergoline as defined by formula (I) of claim 1, as well as their salts, characterized in that a product with the formula (II):

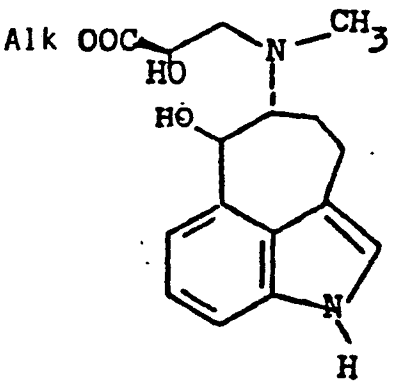

(II)

in which Alk represents an alkyl radical containing from 1 to 4 carbon atoms, is cyclised so as to obtain a product with the formula (III):

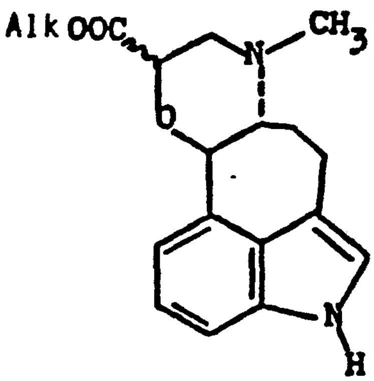

(III)

in which Alk has the significance already indicated, and the wavy line signifies that the substituent is at position 8α or 8β, of which, either the isomer 8β is isolated, or the isomer 8α is epimerized then the acid thus obtained in which the substituent at 8 is at position 8β is esterified,so as to obtain the product with the formula ($I_A$):

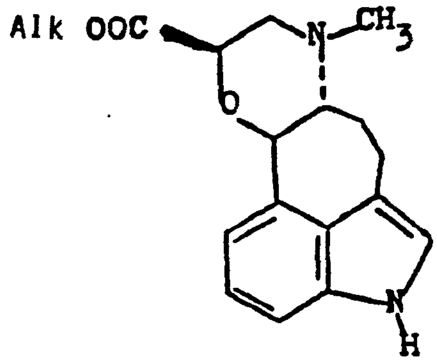

($I_A$)

in which Alk has the significance already indicated, which

- either is isolated and if desired, salified,
- or is demethylized so as to obtain the product with the formula ($I_B$):

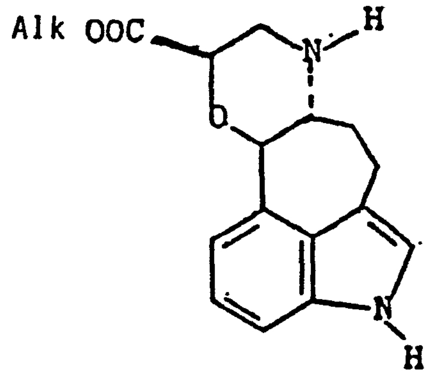

($I_B$)

in which Alk has the significance already indicated, which either is isolated and if desired salified, or is made to react with an alkylation agent so as to obtain a product with the formula ($I_C$):

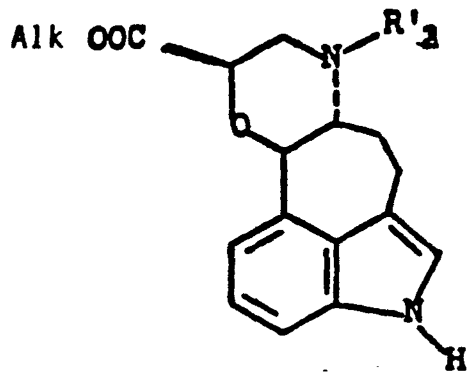

($I_C$)

in which $R'_2$ has the sigificance of $R_2$ already indicated with the exception of hydrogen, which either is isolated, and, if desired, salified, or is made to react with an alkaline amide, then with an alkyl balogenide with the formula (IV)

Hal-R' (IV)

in which Hal represents a chlorine, bromine or iodine atom and R' represents an alkyl radical containing from 1 to 4 carbon atoms, so as to obtain a product with the formula ($I_D$):

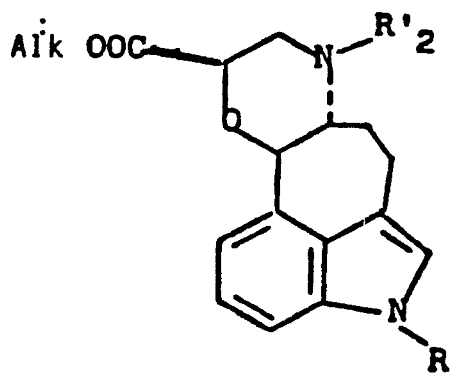

$(I_D)$

in which Alk, R' and $R'_2$ have the significance already indicated, which either is isolated and if desired, salified, or is submitted to the action of a halogenation agent so as to obtain a product with the formula $(I_E)$:

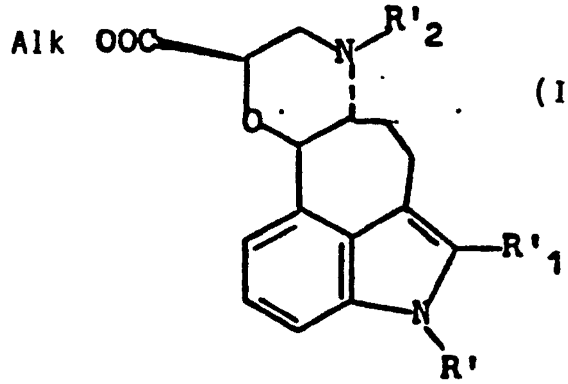

$(I_E)$

in which Alk, R' and $R'_2$ have the significance already indicated, and $R'_1$ represents a chlorine or bromine atom which is isolated and salified if desired, <u>or</u> the said product with the formula $(I_C)$ is submitted to the action of a halogenation agent so as to obtain a product with the formula $(I_F)$:

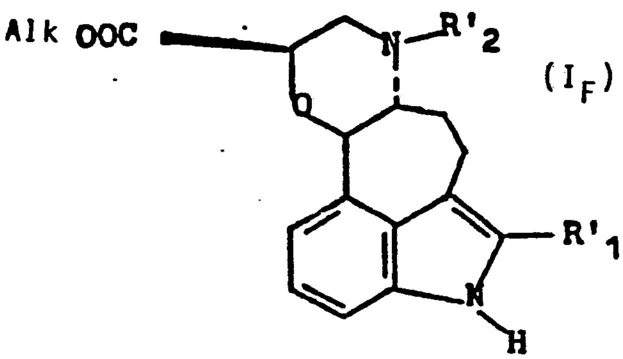

$(I_F)$

in which Alk, $R'_1$ and $R'_2$ have the significance already indicated, which is isolated and if desired, salified, <u>or</u> the said product with the formula $(I_A)$ is saponified so as to obtain a product with the formula $(I_G)$.

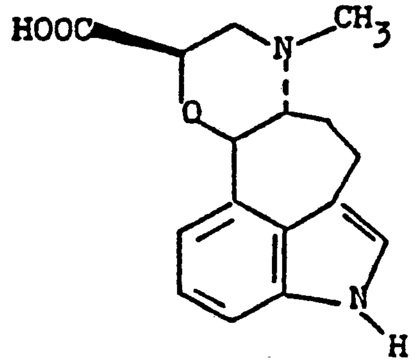

$(I_G)$

which either is isolated and, if desired, salified, or it is made to react with an amine with the formula (V):

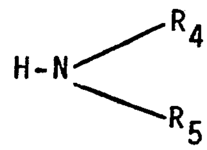

(V)

in which $R_4$ and $R_5$ have the significance already indicated, or with a peptide, so as to obtain a product with the formula $(I_H)$:

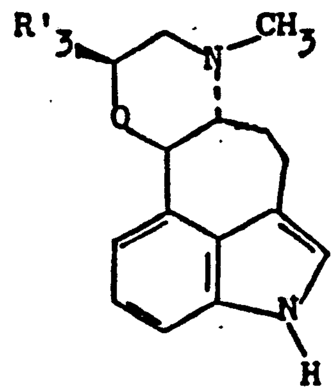

$(I_H)$

in which $R'_3$ represents a carboxy radical amidified by the amine with the formula (V) defined above, which is isolated and if desired, salified, <u>or</u> the said product with the formula $(I_A)$ is reduced so as to obtain a product with the formula $(I_I)$:

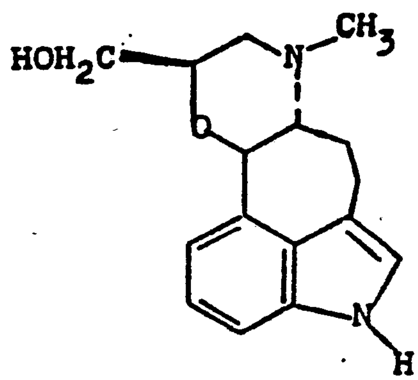

$(I_I$

which is either isolated and, if desired, salified, or is made tc react with methane or paratoluene sulfonyl chloride so as to obtain a product with

the formula (VI):

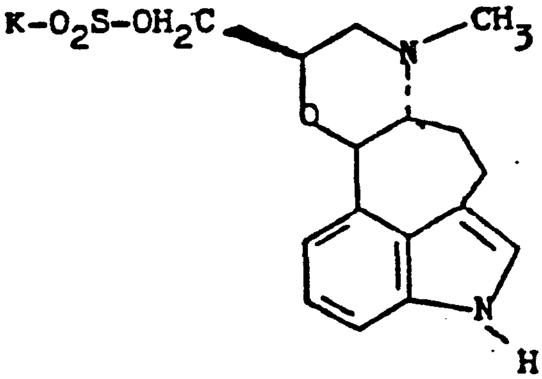

(VI)

in which K represents a methyl or p-tolyl radical, which is made to react with an alkyl mercaptan or with an alkaline cyanide, so as to obtain a product with the formula ($I_J$):

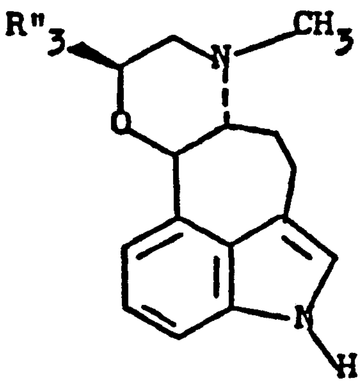

($I_J$)

in which $R''_3$ represents an alkylthiomethyl or cyanomethyl radical which is isolated and if desired, salified, or the product with the formula ($I_A$) is oxidized, so as to obtain a product with the formula ($I_K$)

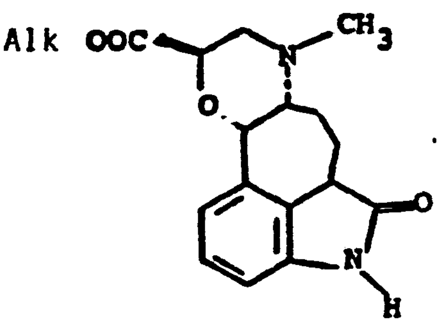

($I_K$)

in which Alk has the significance already indicated, which is either isolated and, if desired, salified, or is reduced So as to obtain a product with the formula ($I_L$):

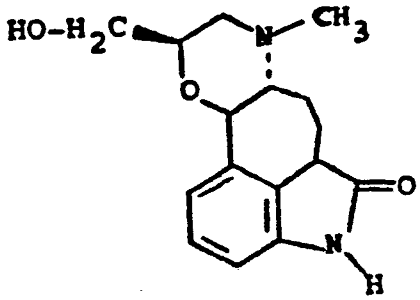

($I_L$)

which is isolated and if desired, salified, then if desired, one or more of the reactions already indicated above for the products with the formula ($I_A$) are carried out on the products with the formulae ($I_F$), ($I_G$), ($I_H$), ($I_1$), ($I_J$), ($I_K$) and ($I_L$), so as to obtain the corresponding products entering in the formula (I), then the products thus obtained are isolated and if desired, salified.

7) Medicaments, characterized in that they are composed of the new derivatives of C-homo-9-oxaergoline, as defined by formula (I) of claim 1, as well as by their salts of addition with pharmaceutically acceptable acids.

8) Medicaments, characterized in that they are composed of the new derivatives of C-homo-9-oxaergoline, as defined in claim 2, as well as by their salts of addition with pharmaceutically acceptable acids.

9) Medicaments, characterized in that they are composed of the new derivatives of C-homo-9-oxaergoline, as defined in claim 3 or 4, as well as by their salts of addition with pharmaceutically acceptable acids.

10) Medicaments, characterized in that they are composed of the derivatives of C-homo-9-oxaergoline, as defined in claim 5, as well as by their salts of addition with pharmaceutically acceptable acids.

11) Pharmaceutical compositions, characterized in that they contain, as active principle, at least one of the medicaments as defined in one of the claims 7 to 10.

12) As new industrial products necessary for the preparation of derivatives answering to the formula (I) of claim 1:

- derivatives with the formula (II):

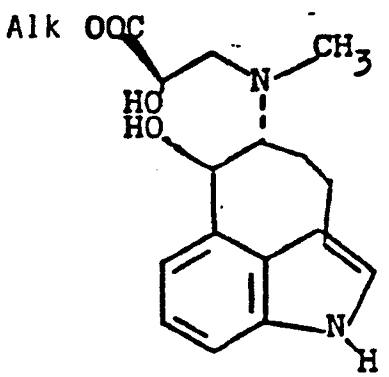

(II)

in which Alk has the significance already indicated in claim 6.

**Claims** for the contracting state AT

1. Preparation process of new derivatives of C-homo 9 oxaergoline, as well as their salts of addition with mineral or organic acids, answering to the general formula (I):

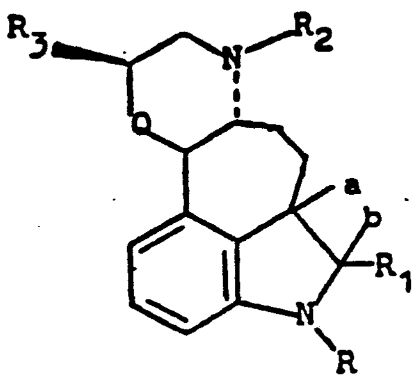

(I)

in which R represents a hydrogen atom or an alkyl radical containing from 1 to 4 carbon atoms, $R_1$ represents a hydrogen, chlorine or bromine atom or forms with b an oxo radical, a forms with b a double bond or a represents a hydrogen atom when b forms an oxo radical with $R_1$, b forms a double bond with a or an oxo radical with $R_1$, $R_2$ represents a hydrogen atom, an alkyl radical containing from 1 to 4 carbon atoms, an aralkyl radical containing from 7 to 12 carbon atoms, or a cycloalkylalkyl radical containing from 4 to 7 carbon atoms, $R_3$ represents a hydroxymethyl, alkylthiomethyl, cyanomethyl or carboxy radical possibly esterified by an aliphatic alcohol containing from 1 to 5 carbon atoms, or amidified by an amine with the formula

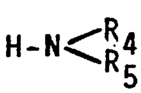

in which $R_4$ represents a hydrogen atom or an alkyl

radical containing from 1 to 4 carbon atoms, and $R_5$ represents an alkyl radical containing from 1 to 4 carbon atoms or $R_4$ and $R_5$ form together with the nitrogen atom a saturated heterocycle possibly containing another heteroatom, which latter can be substituted by an alkyl radical containing from 1 to 4 carbon atoms when the heterocycle contains another nitrogen atom, characterized in that a product with the formula (II):

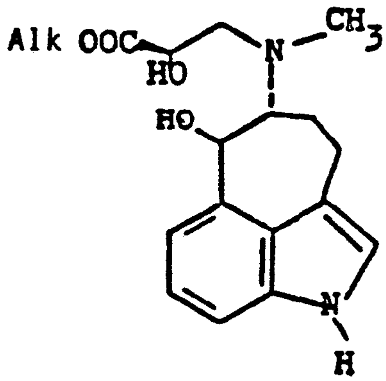

(II)

in which Alk represents an alkyl radical containing from 1 to 4 carbon atoms, is cyclised so as to obtain a product with the formula (III):

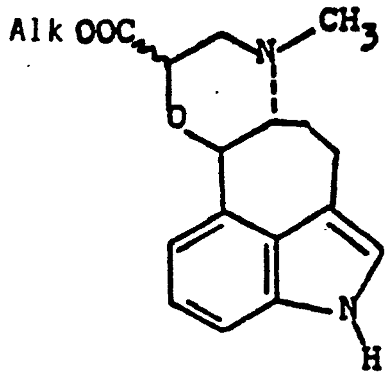

(III)

in which Alk has the significance already indicated, and the wavy liny signifies that the substituent is at position 8α or 8β, of which, either the isomer 8β is isolated, or the isomer 8α is epimerized then the acid thus obtained in which the substituent at 8 is in position 8β, is esterified so as to obtain the product with the formula ($I_A$):

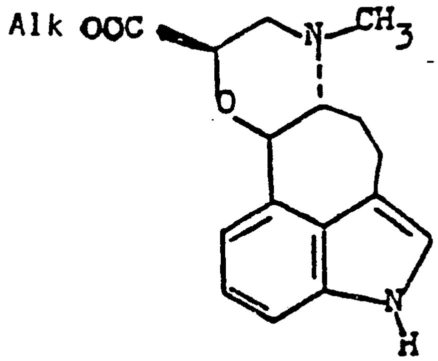

($I_A$)

in which Alk has the significance already indicated, which

- either is isolated and if desired, salified,
- or is demethylized so as to obtain the product with the formula ($I_B$)

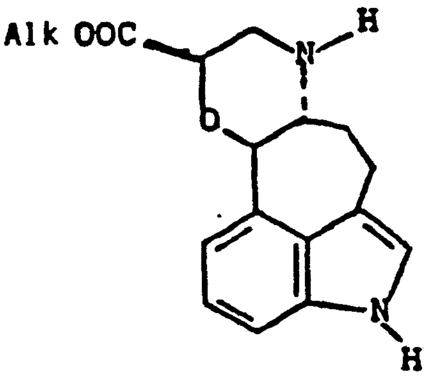

($I_B$)

in which Alk has the significance already indicated, which either is isolated and if desired, salified, or is made to react with an alkylation agent to obtain a product with the formula ($I_C$):

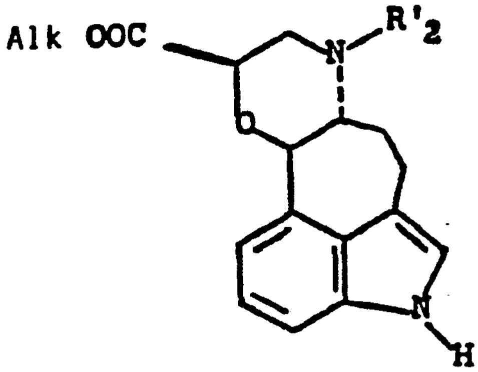

($I_C$)

in which $R'_2$ has the significance of $R_2$ already indicated with the exception of hydrogen, which <u>either</u> is isolated, and if desired, salified, <u>or</u> is made to react with an alkaline amide, then with an alkyl halogenide with the formula (IV):

Hal-R' (IV)

in which Hal represents a chlorine, bromine or iodine atom and R' represents an alkyl radical containing from 1 to 4 carbon atoms, so as to obtain a product with the formula ($I_D$):

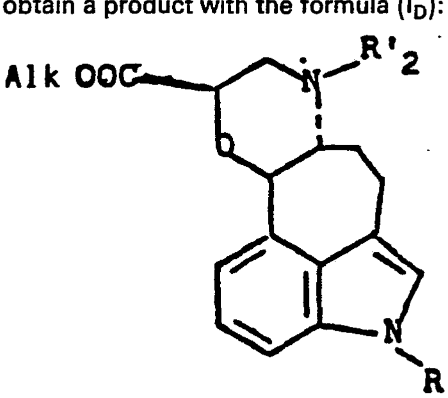

($I_D$)

in which Alk, R' and $R'_2$ have the significance already indicated, which either is isolated and, if desired, salified, or is submitted to the action of a halogenation agent so as to obtain a product with the formula ($I_E$):

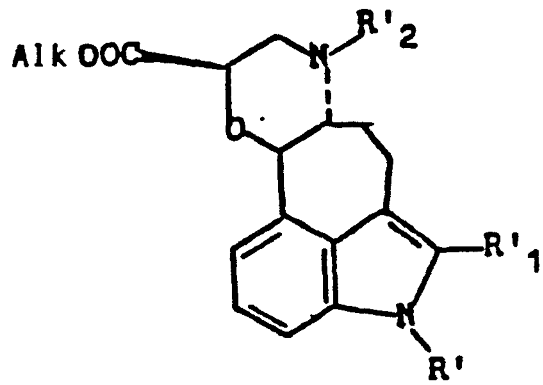

($I_E$)

in which Alk, R' and $R'_2$ have the significance already indicated, and $R'_1$ represents a chlorine or bromine atom which is isolated and salified if desired, <u>or</u> the said product with the formula ($I_C$) is.submitted to the action of a halogenation agent so as to obtain a product with the formula ($I_F$):

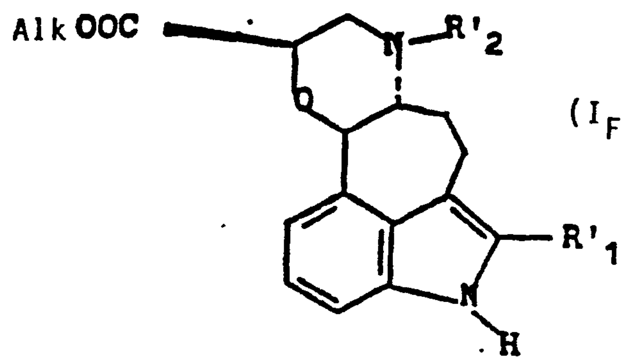

($I_F$)

in which Alk, $R'_1$ and $R'_2$ have the significance already indicated, which is isolated and if desired, salified, <u>or</u> the said product with the formula ($I_A$) is saponified so as to obtain a product with the formula ($I_G$):

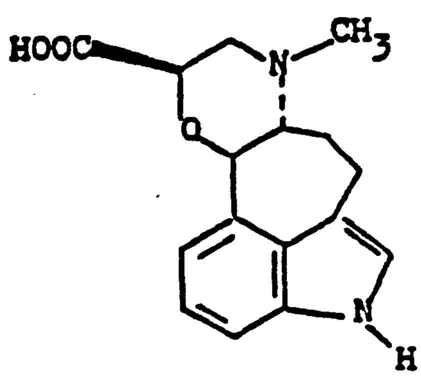

($I_G$)

which is either isolated, and if desired, salified, or is made to react with an amine with the formula (V):

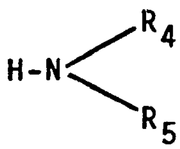

(V)

in which $R_4$ and $R_5$ have the significance already indicated, or with a peptide, so as to obtain a product with the formula ($I_H$):

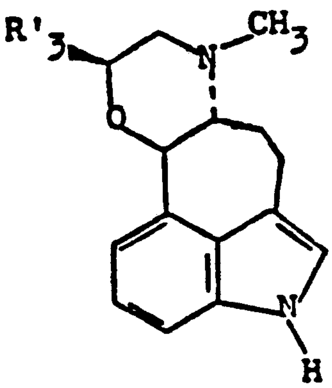

($I_H$)

in which $R'_3$ represents a carboxy radical amidified by the amine with the formula (V) defined above, which is isolated and if desired, salified,

or the said product with the formula ($I_A$) is reduced so as to obtain a product with the formula ($I_I$):

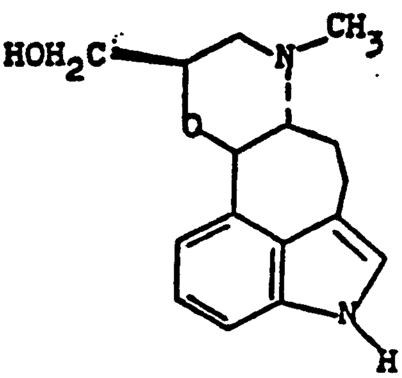

($I_I$)

which either is isolated, and if desired, salified, or is made to react with methane or paratoluene sulfonyl chloride so as to obtain a product with the formula (VI):

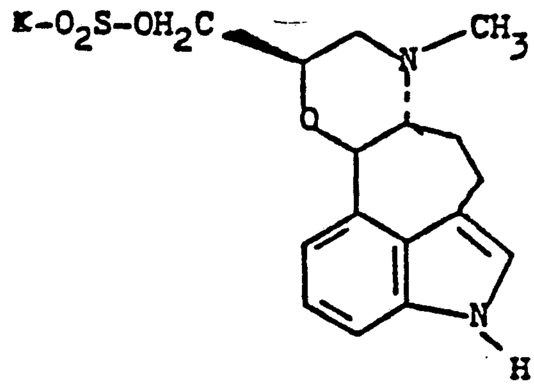

(VI)

in which K represents a methyl or p-tolyl radical, which is made to react with an alkylmercaptan or with an alkäline cyanide. so as to obtain a product with the formula ($I_J$):

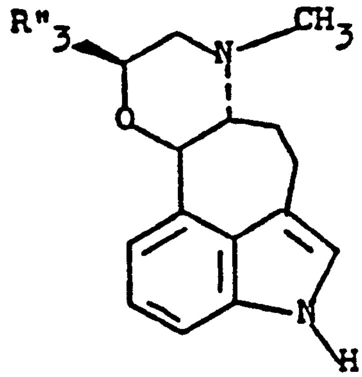

($I_J$)

in which $R''_3$ represents an alkylthiomethyl or cyanomethyl radical which is isolated and if desired, salified,

or the product with the formula ($I_A$) is oxidized, so as to obtain a product with the formula ($I_K$):

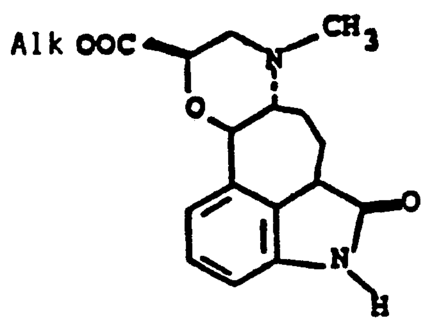

($I_K$

in which Alk has the significance already indicated, which either is isolated and, if desired, salified, or reduced so as to obtain a product with the formula ($I_L$):

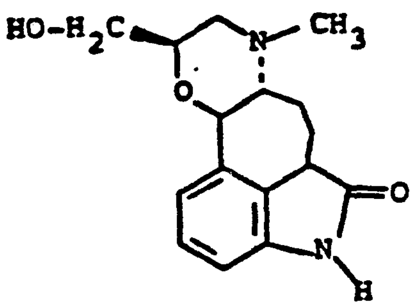

$(I_L)$

which is isolated and if desired salified, then if desired, one or more of the reactions already indicated above for the products with the formula $(I_A)$ are carried out on the products with the formulae $(I_F)$, $(I_G)$, $(I_H)$, $(I_I)$, $(I_J)'$ $(I_K)$ and $(I_L)$, so as to obtain the corresponding products entering into the formula (I), then the products thus obtained are isolated and if desired, salified.

2. Process according to claim 1, for the preparation of derivatives of C-homo-9-oxaergoline answering to formula (I), in which <u>a</u> and <u>b</u> form a double bond, characterized in that a product with the formula (II):

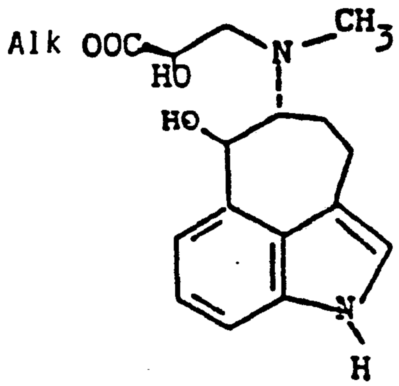

(II)

in which Alk represents an alkyl radical containing from 1 to 4 carbon atoms, is cyclised so as to obtain a product with the formula (III)

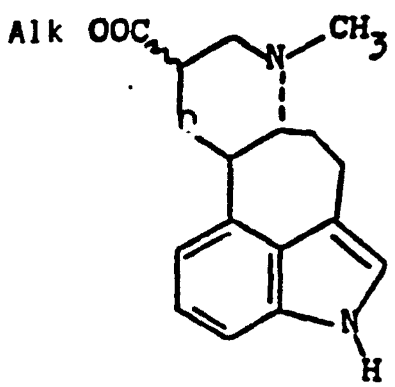

(III)

in which Alk has the significance already indicated, and the wavy line signifies that the substituent is at position 8α or 8β, of which either the isomer 8β is isolated, or the isomer 8α is epimerized then the acid thus obtained in which the substituent at 8 is at position 8β, is esterified,so as to obtain the product with the formula $(I_A)$:

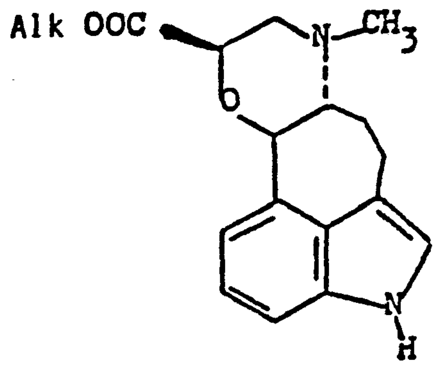

$(I_A)$

in which Alk has the significance already indicated, which

- <u>either</u> is isolated and if desired, salified,
- <u>or</u> is demethylized so as to obtain the product with the formula $(I_B)$:

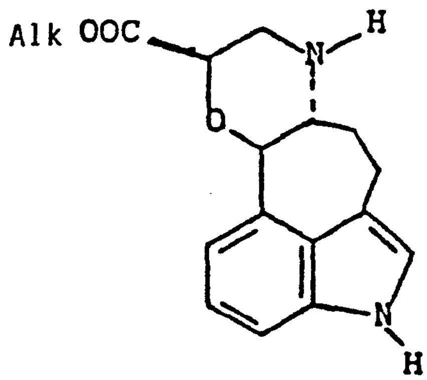

$(I_B)$

in which Alk has the significance already indicated, which either is isolated and if desired, salified, or is made to react with an alkylation agent so as to obtain a product with the formula $(I_C)$.

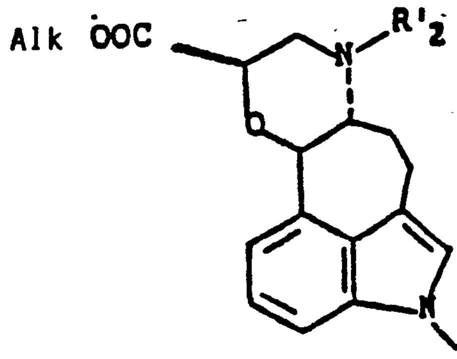

$(I_C)$

in which $R'_2$ has the significance of $R_2$ already indicated with the exception of hydrogen, which <u>either</u> is isolated, and, if desired, salified, <u>or</u> is made to react with an alkaline amide, then with an alkyl halogenide with the formula (IV):

Hal-R' (IV)

in which Hal represents a chlorine, bromine or iodine atom and R' represents an alkyl radical containing from 1 to 4 carbon atoms, so as to obtain a product with the formula $(I_D)$:

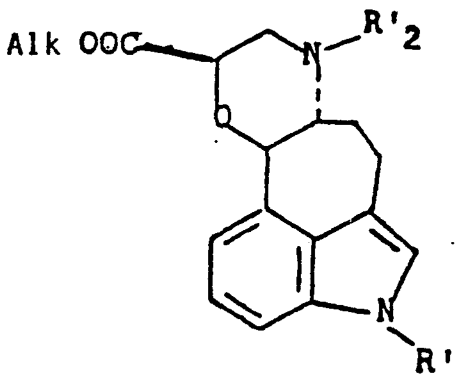

($I_D$)

in which Alk, R' and $R'_2$ have the significance already indicated, which either is isolated and, if desired, salified, or is submitted to the action of a halogenation agent so as to obtain a product with the formula ($I_E$):

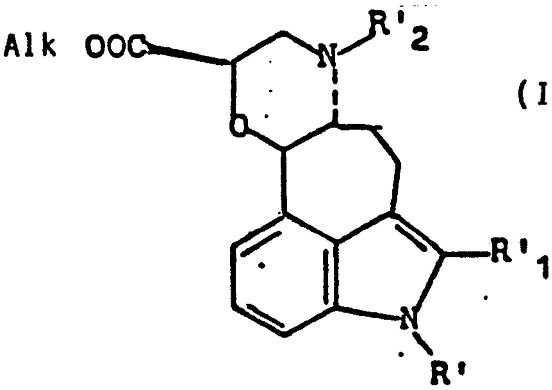

($I_E$)

in which Alk, R' and $R'_2$ have the significance already indicated, and $R'_1$ represents a chlorine or bromine atom which is isolated and salified if desired, or the said product with the formula ($I_C$) is submitted to the action of a halogenation agent to obtain a product with the formula ($I_F$):

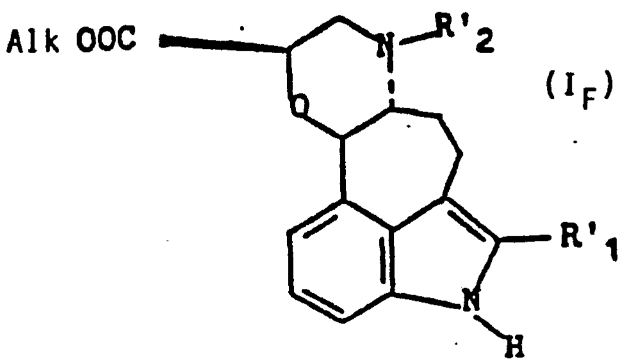

($I_F$)

in which Alk, $R'_1$ and $R'_2$ have the significance already indicated, which is isolated and if desired, salified, or the said product with the formula ($I_A$) is saponified so as to obtain a product with the formula ($I_G$):

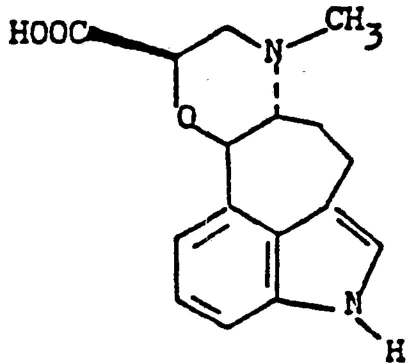

($I_G$)

which either is isolated and, if desired, salified, or is made to react with an amine with the formula (V):

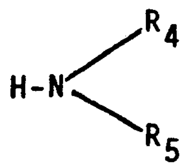

(V)

in which $R_4$ and $R_5$ have the significance already indicated, or with a peptide, so as to obtain a product with the formula ($I_H$):

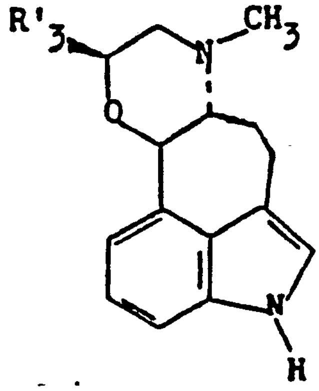

($I_H$)

in which $R'_3$ represents a carboxy radical amidified by the amine with the formula (V) defined above, which is isolated and if desired, salified,

or the said product with the formula ($I_A$) is reduced so as to obtain a product with the formula ($I_I$):

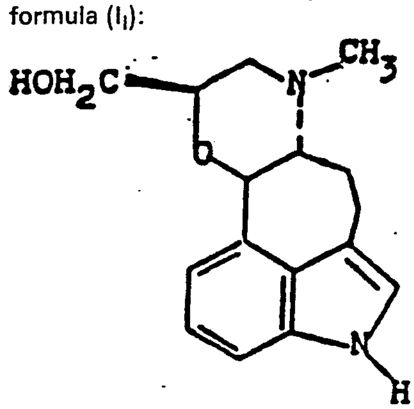

($I_I$)

which either is isolated and, if desired, salified, or is made to react with methaneor paratoluene

sulfonyl chloride so as to obtain a product with the formula (VI):

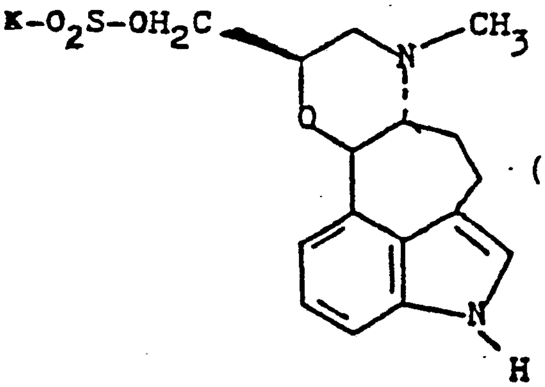

(VI)

inwhich K represents a methyl or p-tolyl radical, which is made to react with an alkyl mercaptan or with an alkaline cyanide, so as to obtain a product with the formula ($I_J$):

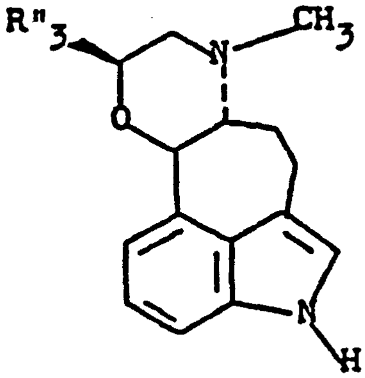

($I_J$)

in which $R''_3$ represents an alkylthiomethyl or cyanomethyl radical which is isolated and if desired, salified, then if desired, one or more of the reactions already indicated above for the products with the formula ($I_A$) are carried out on the products with the formulae ($I_F$), ($I_G$), ($I_H$), ($I_I$), ($I_J$), so as to obtain the corresponding products entering in to the formula (I), then the products thus obtained are isolated and if desired, salified.

3. Process according to claim 2, characterized in that an alkylation agent is used, enabling a $R_2$ radical containing from 1 to 4 carbon atoms to be attached.